(19) Europäisches Patentamt / European Patent Office / Office européen des brevets

(11) **EP 2 921 148 A1**

(12) **EUROPEAN PATENT APPLICATION**
published in accordance with Art. 153(4) EPC

(43) Date of publication:
**23.09.2015 Bulletin 2015/39**

(51) Int Cl.:
*A61F 13/02* (2006.01)    *A61L 15/22* (2006.01)
*A61L 15/44* (2006.01)

(21) Application number: **13854481.2**

(22) Date of filing: **08.11.2013**

(86) International application number:
**PCT/ES2013/070774**

(87) International publication number:
**WO 2014/076336 (22.05.2014 Gazette 2014/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.11.2012 ES 201231750**

(71) Applicants:
  • **Consejo Superior De Investigaciones Científicas (CSIC)**
    **28006 Madrid (ES)**
  • **Fundacion Rioja Salud**
    **26006 Logroño (ES)**
  • **Universidad De Alcalá De Henares**
    **28801 Alcalá De Henares (ES)**

(72) Inventors:
  • **REYES ORTEGA, Felisa**
    **E-28006 Madrid (ES)**
  • **RODRÍGUEZ CRESPO, Gema**
    **E-28006 Madrid (ES)**

  • **AGUILAR DE ARMAS, María Rosa**
    **E-28006 Madrid (ES)**
  • **GONZÁLEZ GÓMEZ, Álvaro**
    **E-28006 Madrid (ES)**
  • **SAN ROMÁN DEL BARRIO, Julio**
    **E-28006 Madrid (ES)**
  • **SOLIS CORREA, Raúl Enrique**
    **E-28006 Madrid (ES)**
  • **GARCÍA HONDUVILLA, Natalio**
    **E-28801 Alcalá de Henares (Madrid) (ES)**
  • **BUJÁN VARELA, Julia**
    **E-28801 Alcalá de Henares (Madrid) (ES)**
  • **CIFUENTES NEGRETE, Alberto**
    **E-28801 Alcalá de Henares (Madrid) (ES)**
  • **MARTÍNEZ RAMÍREZ, Alfredo**
    **E-26006 Logroño (La Rioja) (ES)**
  • **GARCÍA SANMARTÍN, Miren Josune**
    **E-26006 Logroño (La Rioja) (ES)**
  • **STENZEL, Martina**
    **E-26006 Logroño (La Rioja) (ES)**

(74) Representative: **Ungria López, Javier**
    **Avda. Ramón y Cajal, 78**
    **28043 Madrid (ES)**

(54) **DRESSING FOR COMPROMISED WOUND HEALING**

(57)    The invention relates to a bioactive medical product, or dressing, characterised in that it comprises at least an inner layer formed by a hydrogel and an outer layer formed by a biodegradable and bioabsorbable polyurethane, said inner layer being impregnated with an *N*-terminal peptide of 20 amino acids of pro-adrenomedullin and the outer layer being impregnated with nanoparticles of bemiparin encapsulated in a biodegradable copolymer or polymer. The active components can be dosed in a sequential and controlled manner with this composition. The invention also relates to a method for producing the two-layer dressing and to the use thereof in medicine, said dressing being specially designed to treat and promote the healing of wounds, such as compromised wounds and ulcers, particularly in patients with diabetes and reduced blood supply.

FIG. 1

EP 2 921 148 A1

**Description**

**Field of the Invention**

The field in which the present invention is included is the chemical-pharmaceutical industry, as it is a device or material that can be used in the medical field that facilitates and improves the healing of compromised wounds.

**Background of the Invention**

The healing of wounds entails a balance between inflammatory and vascular activity of connective tissue and epithelial cells. Skin wounds heal by two different processes: epithelialisation and contraction. With the use of dressings it is intended to promote the process of epithelialisation over the process of contraction. Healing is faster in a moist environment and the dressings are used to absorb excess fluid or retain it in a wound that would otherwise be dry, in order to achieve a "wet wound environment." Skin lesions caused by vascular insufficiency affect a large number of people and represent a high cost for the health system in developed societies (Jiménez and Jiménez, 2004).

Conventional healing techniques such as suture, cauterization or the use of staples have several disadvantages such as pain and the need to have them removed after tissue regeneration. However, the use of other medical materials such as the dressings has the advantage of not being painful for the patient, it easily adheres to the skin, and their use is easy and simple and they do not need to be removed. In order for the dressing to fulfil this series of advantages it must be biodegradable, biocompatible (it will be in direct contact with the wound so it cannot be toxic), bioabsorbable and have a certain stability (good mechanical properties) so that it can be effective in the process of wound healing.

Currently, most products sold for the healing of compromised wounds are injectable systems that are administered in the lesion itself and/or its surroundings, as described in patent applications WO03/053458A1, WO 2011/002249 or in Zhang et al. (2011). Other systems are formed *in situ* in the area to be regenerated (WO 2011/028031) but the disadvantage of this process is that the final mechanical properties of the system cannot be controlled. Patent application WO2010/120757 describes a three-dimensional single-layer material or system (scaffold) which is formed based on a light-cured polysaccharide (chitosan) containing a protein, which is gelatin, as the bioactive molecule.

Another example of single-layer system is the use of a matrix of collagen for the healing of wounds caused by burns, described by Van Zuijen. Matrices of collagen and sodium hyaluronate cross linked with carbodiimide have been used as single-layer system for tissue regeneration, since they have a good porosity that allows hosting active molecules as growth factors. Sodium hyaluronate is a glycosaminoglycan formed by repeating units of D-glucuronic acid and β-1,3-*N*-acetyl-D-glucosamine-β-1,4. It is the major component of synovial fluid, cartilage tissue and vitreous humour. It has the property to retain large quantities of water and adopting an extended conformation in solution (Mao et al., 2003; Collins and Birkinshaw, 2008; Picard et al., 2009; and Vanderhooft et al., 2009).

There are very few two-layer or multilayer systems for releasing drugs, and the ones that exist have been used in dental implants or coatings (patent application US2011/0038921 A1), but not for the treatment of wounds.

Some of the compounds that are known for promoting the healing of wounds and ulcers are the *N*-terminal peptide of 20 amino acids of pro-adrenomedullin (PAMP; Bergmann et al. 2008), which has angiogenic, reepithelialisation and antimicrobial properties; and bemiparin (low molecular weight heparin; Martinez-González and Rodríguez, 2010; Rullán et al., 2008), which is a sulfonated polysaccharide belonging to the family of glycosaminoglycans that acts as a thrombotic and/or restenotic modulator and it is also able to form active complexes with growth factors (VEGF and FGF among others), increasing the biological activity of said factors (Capila and Linhardt, 2002).

The use of nanoparticles as drug carrier systems has been widely used in recent decades for being an efficient drug encapsulation and delivery system and of easy administration as an injectable system in patients (Ríos, 2003; Lassalle and Ferreira, 2007; and Mundargi et al., 2008). The use of biodegradable polymers derived from lactic and glycolic acids known as PLGA and its derivatives is widespread since it is an exemplary material due to its biodegradable and biocompatible properties and for being able to efficiently encapsulate a variety of molecules (proteins, peptides, genes, vaccines, antigens, growth factors, etc. (Capila, I., and R. J. Linhardt. 2002. Heparin-Protein Interactions. Angewandte Chemie International Edition 41 (3): 390-412). Matrices based on EUDRAGIT (Ceballos et al., 2005; Dillen et al., 2006; and Moustafine et al., 2006) are quite attractive in the pharmaceutical business due to their high chemical stability, good compactness and availability of a wide variability of products on the market that have different chemical properties. Numerous studies have been published on nanoparticles prepared with PLGA and EUDRAGIT to encapsulate heparin, with the object of improving the stability of said drug in the body (Jiao et al., 2002; Hoffart et al., 2003; Chung et al., 2006; and Hoffart et al., 2006).

Currently, dressings including PAMP in their structure and that release it in the healing processes, or composite materials that release heparin together with other active components, in a sequential and controlled manner, have not been described.

Sequential and controlled dosing of both active components (PAMP and heparin) has been achieved with the system described here, thus allowing revascularization and healing of compromised wounds, particularly in patients with diabetes and reduced blood supply. Thus, a medical product is proposed in this paper, which is a dressing (a gauze, a plaster...), for use in regeneration of skin compromised wounds, compromised wound meaning a wound having compromised healing, which refers to abnormal scarring, that is, scarring that may be affected (usually delayed) by a pathology such as diabetes or ischemic processes. The dressing consists of at least two bio-functionalized layers able to improve the healing of wounds and ulcers, particularly in cases where the process is hampered such as in diabetic patients with reduced blood flow and elderly people. For this, biodegradable and resorbable polymeric composite materials able to act as support and controllably release the active components were prepared.

## General description of the Invention

The present invention is directed to a bioactive medical product, which is a dressing, characterised in that it comprises at least:

- one inner layer formed by a hydrogel consisting of a combination of sodium hyaluronate and gelatin, and being cross-linked (stabilized) with at least one crosslinking agent, and
- one outer layer formed by a block copolymer type $(A_nB_m)_x(C_oD_p)_y$ which is biodegradable and bioabsorbable polyurethane,

the inner layer being impregnated with an *N*-terminal peptide of 20 amino acids of pro-adrenomedullin (PAMP), and the outer layer with nanoparticles of bemiparin encapsulated in a biodegradable copolymer or polymer.

This multilayer dressing is biodegradable, bioabsorbable and biocompatible, having optimal mechanical properties for sequential and metered release of the bioactive compounds it incorporates, thus being particularly useful for revascularization and regeneration of wounds, being of particular interest for compromised ulcers on the skin. The dosage of the bioactive components (PAMP and nanoparticles containing bemiparin) occurs in a programmed manner in time, according to the biodegradative behaviour of each of the layers. When configured as described, with the PAMP in the hydrogel layer and the bemiparin on the polyurethane layer, the wound healing process is promoted by an epithelialisation process and not by wound contraction. Likewise, the arrangement of the layers is what allows a sequential release.

Hydrogels formed by hyaluronic acid-gelatin have the advantage of providing a moist environment suitable for cell migration and proliferation, and have hydrophilic properties, offering a hydration balance maintained in time necessary for optimal healing of lesions. On the one side, the gelatin has lysine or glutamine residues in its chemical structure having free amino groups that can act as crosslinking points and also allows obtaining a much more stable gel. Meanwhile, sodium hyaluronate has the property of retaining large amounts of water and adopting an extended conformation in solution, providing good moisturizing properties to the hydrogel. In turn, the crosslinking allows obtaining a hydrogel with good mechanical and thermal stability. The hydrogel is degraded and absorbed over time, not being necessary to remove it.

The polyurethane base, which isolates and protects the rest of the dressing from the outside is biodegradable, and allows release of said molecule. Segmented polyurethanes are block copolymers which consist of two types of segment: soft and rigid. The rigid segments include connections of diisocyanate (aliphatic or aromatic) and low molecular weight diamine or diol; the latter are called chain extenders. The combination of both types of segments form a copolymer of the type $(A_nB_m)_x(C_oD_p)_y$, $A_nB_m$ being the soft segment and $C_oD_p$ the rigid segment. There is a certain degree of immiscibility between the rigid and soft segments of segmented polyurethane; this means that although the polyurethanes are isotropic, microscopically they are not structurally homogeneous. There is a certain degree of mixing between these two types of segments although phase separation of the two segments occurs, producing a structure consisting of micro domains of rigid segments dispersed in a matrix of soft segments. This structure with micro domains exhibited by polyurethanes is responsible for its excellent mechanical properties and it also contributes to their biocompatibility. This layer is hydrophobic in nature and has slow degradation also stimulating revascularization of damaged tissue and it protects against infectious agents. Thanks to this appropriate dosage over time, the bioactive agent PAMP acts on the outermost layers of the skin, stimulating blood vessel formation in the outermost surface of the dermis. Applying the peptide directly on the wound is not appropriate since it migrates into the deep layers of the skin and stimulates angiogenesis in the dermis where it is not as necessary.

The bio-functionalization of the dressing is carried out on one side with an *N*-terminal peptide of 20 amino acids of pro-adrenomedullin (PAMP) having angiogenic, reepithelialising, antimicrobial and antibacterial properties, which are useful qualities in the healing process by protecting against infectious agents. This peptide is expressed in human skin naturally, so it has been shown as an ideal molecule for bio-functionalizing a medical product intended to promote the healing of wounds and ulcers. Since it is incorporated into the structure of the inner layer it is released directly to the underlying tissue. Furthermore, this molecule is included in a hydrogel that is biodegradable (it is degraded and reabsorbed), which ensures sustained release over time.

On the other side, the dressing is bio-functionalized with bemiparin, a low molecular weight heparin that acts as a modulator of thrombosis and/or restenosis, and that is also capable of forming active complexes with growth factors (VEGF and FGF, among others) increasing the biological activity of said factors. Thus the healing of compromised wounds in the long term is ensured. Heparins showing values between 3 and 6 kDa and chain lengths of 12 to 18 saccharide units are known as low molecular weight heparins. Bemiparin in particular is obtained by chemical degradation of heparin and has a molecular weight of about 3.6 kDa.

Bemiparin is encapsulated in nanoparticles which may have different polymer formulations, preventing premature interaction of this type of heparin with the peptide PAMP. The use of nanoparticles as bemiparin carrier systems confer greater stability to this compound compared to its free form, maintaining its biological activity and making it dose dependent. Thus, it has been shown that the stability of the encapsulated bemiparin is greater than in its free form as the carrier nanoparticles maintain the activity of bemiparin. The bioactivity of these nanoparticles loaded with bemiparin has been shown from a cell proliferation assay modulated by the formation of a ternary complex between the fibroblast growth factor (bFGF), its membrane receptor FGFR-1c and bemiparin (Example 4). The bFGF is comprised within the family of fibroblast growth factors (FGF). *In vivo,* FGFs can induce the formation of mesoderm in developing Xenopus embryos, which have a potent angiogenic activity (ES 2 204 886 T3). BFGF forms a specific ternary complex with heparin and its membrane receptor, so that it modulates cellular response (Bos et al., 1999; and Tanghetti, 2002).

In short, the present document describes novel bemiparin carrier systems together with other bioactive agents, which have a chemical structure similar to EUDRAGIT but that improve the diffusion of the drug in the polymer matrix, thus facilitating its controlled release.

A second object of the invention is a method for the preparation of the medical product described herein, comprising at least the following steps:

- adding the nanoparticles of bemiparin to the polyurethane layer by impregnation with an aqueous dispersion of said nanoparticles;
- hydrating the hydrogel layer and depositing said layer onto the polyurethane layer previously impregnated with the nanoparticles of bemiparin;
- adding the *N*-terminal peptide of 20 amino acids of pro-adrenomedullin to the hydrogel layer by means of aqueous solution; and
- sealing the product obtained from the two layers.

A third object of the present invention would consist of the dressing described, in all its variants, for the use thereof in medicine. This specification thus includes both the use of the medical product in medicine and a method of medical treatment comprising applying the medical product on an outer part (skin) of the body of a patient. Likewise, it includes the use of a layer (internal) formed by a hydrogel consisting of a combination of sodium hyaluronate and gelatin, the hydrogel being cross-linked (stabilized) with at least one crosslinking agent, and which is impregnated with an *N*-terminal peptide of 20 amino acids of pro-adrenomedullin; and a second layer (external) formed by a block copolymer type $(A_nB_m)_x(C_oD_p)_y$ which is biodegradable and bioabsorbable polyurethane containing nanoparticles of bemiparin encapsulated in a biodegradable copolymer or polymer for the manufacture of a dressing for the use thereof in medicine. It must be understood within the scope of the present invention that the patient may be an animal or a human being. In particular, but without being a limitation to its use, the product can be used with patients with diabetes and reduced blood supply.

Similarly, the present invention relates to a kit for coating skin wounds and ulcers, comprising the medical product described above.

### Detailed description of the invention

The medical product object of protection consists of a biodegradable system formed by two layers of different structure and nature to act as a targeted and controlled delivery system of bioactive compounds. Said product, as mentioned above, is a dressing that can encompass a wide variety of materials such as bandages, gauze, plasters, etc.

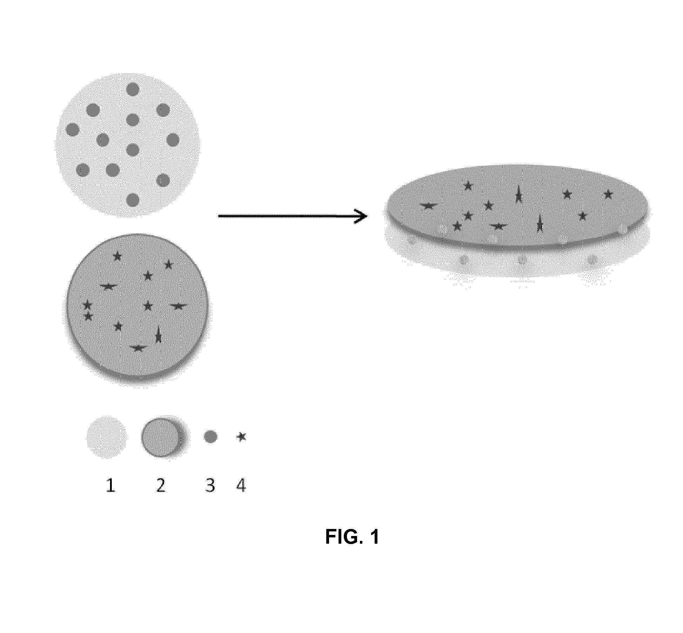

The bioactive agents are released quickly or slowly depending on whether they are incorporated in the inner or outer layer of the medical product (Fig. 1).

The crosslinking agent of the hydrogel is preferably selected from the group consisting of genipin, sodium tripolyphosphate, sodium glycerol phosphate and salicin. Even more preferably, the hydrogel is stabilized with genipin, which provides it with stability and high hydrophilicity. Preferably, the concentration of the crosslinking agent in the hydrogel is comprised between 0.5% and 10% by weight with respect to the polymer mixture, being still more preferred a concentration of 1 % by weight with respect to said mixture.

In a preferred embodiment, the hydrogel consists of 60% by weight of gelatin and 40% by weight of sodium hyaluronate over the polymeric mass formed by the hydrogel, although this proportion may vary in other values and it may be, for example, the opposite: 40% by weight of gelatin and 60% of sodium hyaluronate. However, it must be borne in mind that mechanical consistency varies with the proportion; the higher the proportion of gelatin, the more strength the hydrogel formed will have and the higher the hyaluronate content, the more flexible the end hydrogel is. In another preferred embodiment, the hydrogel additionally incorporates at least one polymer selected from collagen, chitosan and dextran. The hydrogel can be prepared by mixture of aqueous solutions of the two components, a mixture to which the crosslinking agent is subsequently added.

Preferably, the polyurethane, of formula $(A_nB_m)_x(C_oD_p)_y$, has a molecular weight comprised between 3000 and 40000 Daltons, including both limits, being more preferably 19000 Daltons. More preferably, it consists of a soft segment $(A_nB_m)$ formed from polycaprolactone diol (PLC) and pluronic-L61 (PL61) and a rigid segment $(C_oD_p)$ consisting of a poly(tetramethylene glycol ether) (PTMG) and L-lysine diisocyanate selected from methyl, ethyl, propyl, isopropyl, butyl and isobutyl. Preferably it is methyl L-lysine diisocyanate. In a preferred embodiment, the polyurethane is a block co-polymer type $(A_nB_m)_x(C_oD_p)_y$ where n and m are comprised between 0.2 and 0.8, where m + n = 1 and preferably m=n=0.5, o=2 and p=1. X and y are preferably comprised between 0.2 and 0.8, where x + y = 1 and preferably x=y=0.5. A= poly(caprolactone diol), B= pluronic-L61, C= methyl L-lysine diisocyanate) and D= poly(tetramethylene glycol ether).

In the synthesis of polyurethanes, the use of macrodiols with high chain length dispersion has a significant influence on the morphology and phase separation. Therefore, the need for hydrolyzable polyols with uniform chain length, controlled functionality and suitable molecular weight requires careful synthesis and characterization of the products. The chain length influences the crystallinity of the polyester, while the uniformity has an important influence on the degree of phase separation in the polyurethane systems.

The use of L-lysine diisocyanate (LDI) in polyurethane formulation is due to its biodegradability and biocompatibility (Rechichi et al., 2008; and Han et al., 2011), which properties result in it being used in many biomedical applications.

Preferably, the polymer that encapsulates the bemiparin in the nanoparticles is selected from PLGA, EUDRAGIT and CHITOSAN, whereas if it is a copolymer, the latter is in a preferred case a methacrylic amphiphilic block copolymer formed by poly(methyl methacrylate-*b*-[2-(methacryloyloxy)ethyl] trimethylammonium) (poly(MMA-*b*-MAETMA) with a molecular weight $M_w$ between 8000 and 100000, 80000 being preferred. The molar composition can vary between (98:2) and (50:50), (98:2) being preferred.

Preferably, the nanoparticles that encapsulate the bemiparin have an average size comprised between 20 and 1000 nm, said size being more preferably comprised between 50-300 nm, including both limits. The size of the nanoparticles was measured by dynamic light scattering, Zetasizer model Nano ZS by Malvern Instruments.

The medical product can optionally include other layers, such as an adhesive layer to secure it to the body where it is applied with the purpose of healing the wound or ulcer. It can also optionally include one or more additional active agents in each of its layers, interchangeably, such as antibiotics, antiinflammatories or growth factors, among others. Active agent means a product to which beneficial effects on the human being are attributed.

Regarding the process for obtaining the dressing, it should be noted that the synthesis of the block copolymers was preferably carried out by radical polymerization controlled by reversible addition-fragmentation chain transfer (RAFT), which technique has been recently described by Keddie et al. (2012) and Gregori and Stenzel (2012). This technique is one of the most powerful of the living radical polymerization, since it can be used to polymerize virtually all the common monomers and it has been successfully used in homogeneous systems, including water (MacCormick and Lowe, 2004; and Lowe and McCormick, 2007), and heterogeneous systems (Lansalot et al., 2002; and Biasutti et al., 2005). RAFT can be used for the synthesis of block copolymers with low polydispersity indices and high efficiency (Stenzel et al. 2004; Garnier and Laschewsky, 2005; and Yusa et al., 2005). In addition, RAFT can be used for the synthesis of more complex structures (Barner-Kowollik et al., 2003; and Gregori and Stenzel, 2012).

Preferably, the aqueous dispersion of nanoparticles contains a percentage by weight of said nanoparticles comprised between 1% and 25% by mass with respect to the polymeric mass (polyurethane) including both limits, being still more preferably 5% by mass with respect to the polymeric mass. That is, a percentage of 5% of nanoparticles in the solution means that for every 60 mg of polyurethane there are 3 mg of nanoparticles. Each polyurethane sample or disc of 2 cm of diameter weighs 60 mg and thus 3 mg of nanoparticles loaded with bemiparin are added. That gives 5% by mass of the nanoparticles with respect to the mass of polyurethane. In this example, the solution of nanoparticles used has a concentration of 6 mg/ml, 0.5 ml of which is added onto the polyurethane disc, thereby obtaining a load of 3 mg of nanoparticles per disc (or sample).

On the other side, the aqueous solution containing *N*-terminal of 20 amino acids of pro-adrenomedullin and which is added to the hydrogel has a concentration of said peptides PAMP comprised between 1 fmol to 1 mmol PAMP/disc, being more preferably of 1 nmol PAMP/disc. When it is stated that one nmol of PAMP is added to the layer of the hydrogel it means that for every disc (layer) of hydrogel there is 1 nmol of PAMP. For this, for example, a 2 micromolar solution of PAMP is prepared and 0.5 ml of this is taken to load the hydrogel so that the net concentration per hydrogel layer is of 1 nmol of PAMP.

Preferably, after adding the nanoparticles to the polyurethane layer, the mixture is kept for 24 hours controlling the saturation of atmospheric water, which promotes the diffusion of the particles through the film. Also preferably, the solvent can be left to evaporate at room temperature. Also preferably, the hydrogel is hydrated with PBS for a time comprised between 0.5 and 4 hours, more preferably 2 hrs, and then is deposited on the polyurethane film loaded with the nanoparticles of bemiparin. Preferably, the combination of the layers is left for two hours for promoting swelling thereof and additionally 24 hours of contact. In a preferred embodiment, the dressing is sealed between two layers of parafilm to prevent complete evaporation of the solvent. Even more preferably, the dressing is sealed after hydration of both layers, by adding a small amount of distilled water (0.2-0.5 ml) and then contacting said layers and keeping them for 24 hrs under saturated water vapour atmosphere, for promoting sealing.

The medical product object of the invention may be used in a preferred embodiment for the treatment and healing of wounds, more preferably compromised wounds, and ulcers. Particularly and preferably, it is applicable to patients with diabetes, reduced blood flow or elderly people.

**BRIEF DESCRIPTION OF THE FIGURES**

**Figure 1.** Diagram of the two-layer dressing synthesized with the bioactive agents (PAMP and bemiparin). 1) Polyurethane layer, 2) Hydrogel of gelatin-sodium hyaluronate, 3) Nanoparticles loaded with bemiparin, 4) PAMP.

**Figure 2.** a) Diagram of the formation of the hydrogel layer consisting of gelatin and sodium hyaluronate cross linked with genipin, b) Photograph of the xerogels and the hydrogels in water.

**Figure 3.** ATR-FTIR spectra of the gelatin-sodium hyaluronate system cross linked with 5% genipin at different reaction time(s).

**Figure 4.** Degree of hydration achieved at equilibrium by the gels cross linked with genipin at different pH and with different concentrations of genipin (% w/w genipin/polymer).

**Figure 5.** Dynamic time scan of the gelatin-sodium hyaluronate system cross linked with different concentrations of genipin.

**Figure 6.** Frequency scans of the gelatin-sodium hyaluronate system using different concentrations of genipin as crosslinking agent.

**Figure 7.** Synthesis scheme of the chain extender (soft segment).

**Figure 8.** Synthesis scheme of the rigid segment.

**Figure 9.** Synthesis scheme of the SPU321-LDI-[50PCL-diol-50PL61].

**Figure 10.** Thermogram obtained by DSC at 5 °C/min under nitrogen atmosphere.

**Figure 11.** ATR-FTIR spectrum of the SPU321-LDI-[5OPCL-diol-50PL61] synthesized.

**Figure 12.** $^1$H-RMN spectrum of the SPU321-LDI-[50PCL-diol-50PL61] prepared.

**Figure 13.** Thermogravimetric analysis of the SPU321-LDI-[50PCL-diol-50PL61].

**Figure 14.** Degree of hydration of the SPU321-LDI-[50PCL-diol-50PL61] in water.

**Figure 15.** Polymerization scheme of the MMA with the transfer agent CPADB.

**Figure 16.** Synthesis scheme of the block copolymers via RAFT.

**Figure 17.** Evolution of the distribution of molecular weight of the PMMA with time and conversion.

**Figure 18.** $^1$H-RMN spectrum of the PMMA obtained by RAFT polymerisation.

**Figure 20.** $^1$H-RMN spectrum of the block copolymer 86/14 p(MMA$_n$-*b*-MAETMA$_m$) in DMSO-d6.

**Figure 21.** Scheme of formation of the nanoparticles by the method of multiple emulsion. 1) Unimicelar System, 2) Multimicelar System, 3) NP loaded with bemiparin, 4) Image obtained by TEM of the nanoparticulate system consisting of the block copolymer RAFT 86/14 p(MMA$_n$-*b*-MAETMA$_m$) loaded with bemiparin, 5) w/o emulsion, 6) w/o/w emulsion.

**Figure 22.** Images obtained by SEM of the various systems of nanoparticles: a) EUDRAGIT, b) PLGA, c) CHITOSAN, d) 86/14 p(MMA$_n$-*b*-MAETMA$_m$), and) 92/8 p (MMA$_n$-*b*-MAETMA$_m$) and f) 98/2 p(MMA$_n$-*b*-MAETMA$_m$).

**Figure 23.** EDAX analysis carried out at 15 keV of the block copolymers p(MMA$_n$-*b*-MAETMA$_m$), a) 86/14, b) 92/8 and c) 98/2.

**Figure 24.** FTIR spectrum of the bemiparin (solid line), of the PLGA (long dash), of the nanoparticulate system PLGA-bemiparin (short dash).

**Figure 25.** Release profile of the bemiparin at pH= 7.4 at 37 °C over time, HPLC-UV-Vis.

**Figure 26.** Release profile of the bemiparin at pH= 10 at 37 °C over time, HPLC-UV-Vis.

**Figure 27.** Scheme of formation of the ternary complex between the bemiparin, the FGF-2 and its corresponding membrane receptor (FGFR- Ic).

**Figure 28.** Proliferation assay BaF32 with the different systems of NP using different concentrations of bemiparin.

**Figure 29.** SEM images of the nanoparticles of the 98/2 p(MMA-b-MAETMA) NP system on the PU film (a, b) inside the PU film, (c) on the top surface of the PU film, (d) on the bottom surface of the PU film.

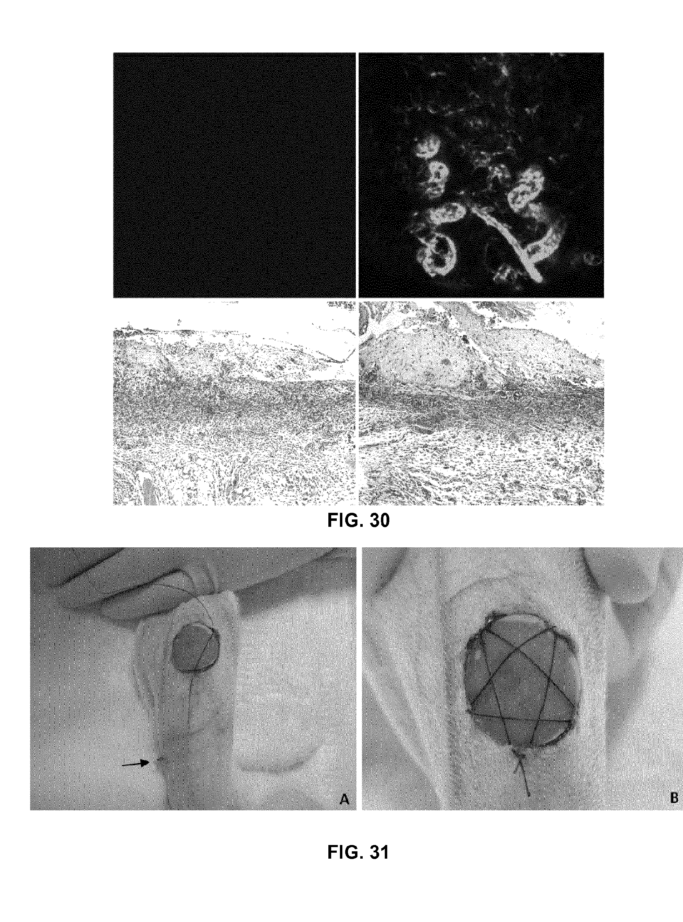

**Figure 30.** Images obtained by confocal and brightfield microscopy a) micrography of the control two-layer system (formed by the hydrogel and the polyurethane layer), b) micrography of the two-layer system containing 1 nmol PAMP in the HG and 5% w of the 98/2 p(MMA-*b*-MAETMA) NP system in the polyurethane layer, after being grafted for 5 days under the skin of the mice, c) image of the transverse histological section of the tissue stained with hematoxylin-eosin after 14 days of implantation of the control two-layer system, d) image of the transverse histological section of the tissue with implanted SPU + NP + BEMI + HG + PAMP two-layer system.

**Figure 31.** Attachment system of the two-layer discs in an ischemic animal. A non-absorbable suture is used continuously (A) fixed at five points, forming a five-pointed star (B). The incision due to the induction of the ischemia is shown in A (arrow).

**Fig. 32:** Morphometric analysis of the processes of contraction and reepithelialisation. The data were obtained from of the measurements taken on the wounds after killing the animals. A greater contribution of the reepithelialisation in the closing is observed in the groups treated with the systems loaded with PAMP. The data are expressed as percentage of the initial area of the defect covered by each process.

**Figure 33.** Immunohistochemistry against $\alpha$SMA. A greater number of sectioned vessels is observed (arrow tips) in the ischemic group +Bemi/+PAMP (A) against the -Bemi/-PAMP group (B). 200x magnification images. (C) Values obtained in the counts of the six groups, expressed as the number of vascular sections by field observed at 200x magnification.

## EXAMPLES

The dressing object of protection, as well as its effects and advantages in the medical field are illustrated next by means of several examples which should not be construed as limiting thereof in any case. This medical product containing bioactive molecules was tested in two animal models: mice and rabbits. Normoxic and ischemic rabbits, with a degree of ischemia 3 have been used to simulate and compare patients without impaired healing with those with diabetes or circulatory problems. The mice were genetically engineered to express the green fluorescent protein (GFP) in the blood vessels.

### *Example 1: Gelatin-sodium hyaluronate hydrogels cross linked with genipin.*

Materials

Pork gelatin (GE) A type was obtained from Sigma-Aldrich, the oral grade sodium hyaluronate (HA) with molecular weight of 100 kDa was obtained from Bioibérica, and the genipin was obtained from Challenge Bioproducts CO. All the reagents were used without prior purification.

Methods

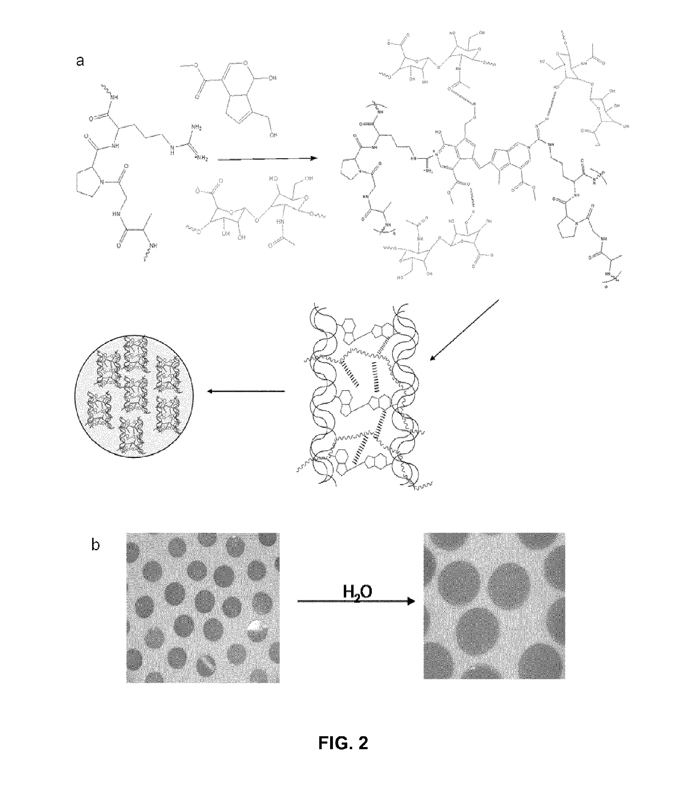

The preparation of the hydrogels of gelatin and sodium hyaluronate has been carried out using various crosslinking agents with the purpose of obtaining chemically or physically cross linked systems (Fig. 2). The hydrogels were obtained by mixing both biopolymers, which were cross linked by using different concentrations (0.5, 1, 2 and 5 % with respect to the polymer mixture) of salicin, genipin, tripolyphosphate and glycerol phosphate. The hydrogels were prepared by casting a mixture of aqueous solutions of both components: 60% gelatin (100 mg/ml) and 40% sodium hyaluronate (50 mg/ml). The crosslinker was added to the mixture and it was rapidly placed in a Teflon mould where the water was allowed to evaporate at room temperature.

Thermogravimetric tests (TGA), rheological and swelling studies of the hydrogels were carried out to test its dynamic mechanical strength and stability.

Characterization

The characterization by ATR-FTIR was carried out using two $CaF_2$ crystals of 2 mm thick and 25 mm diameter. Perkin-Elmer Spectrum One equipment was used, carrying out a scan from 4000 to 600 cm$^{-1}$. 32 scans with a resolution of 4 cm$^{-1}$ were carried out.

The thermogravimetric analysis (TGA) was carried out on TGA Q500 (TA) equipment between 25 and 600 °C under nitrogen atmosphere at 10 °C/min.

The rheology measurements were carried out on a controlled stress oscillatory rheometer from TA Instruments model ARG2, using parallel plate geometry. The samples in dissolution were measured using a top methacrylate plate and 40 mm in diameter. For the samples in gel state a steel top plate of 20 mm in diameter was used.

Results and discussion

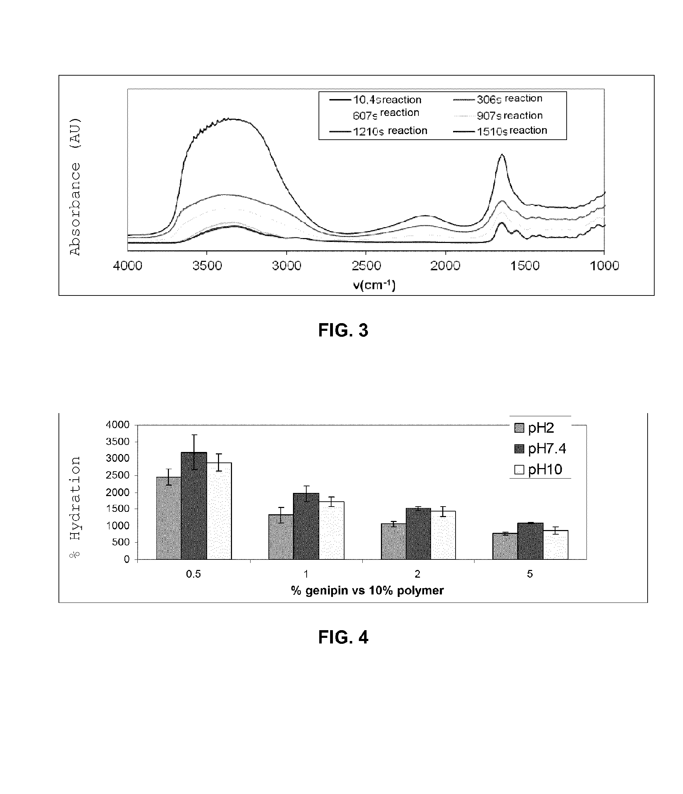

The process of the crosslinking reaction can be observed (Fig. 3) through the decrease of intensities of the bands characteristic of the functional groups ($V_{OH}$ = 3600 cm$^{-1}$, $V_{NH2}$ = 3400 cm$^{-1}$, $V_{CO}$ = 1660 cm$^{-1}$) of the hydrogel in the ATR-FTIR spectrum. When the crosslinking occurs via hydrogen bonds between the OH, COOH and NH$_2$ groups, an enlargement and a reduction in the intensity of the characteristic bands is observed. Furthermore, by increasing the reaction time, the occurrence of a second signal from a carbonyl group is observed ($V_{CO}$= 1620 cm$^{-1}$) that is due to the chemical crosslinking that occurs between the gelatin and the genipin that results in new carbonyl bonds with a chemical environment different than the original, which have uncrosslinked polymers ($V_{CO}$= 1660 cm$^{-1}$).

*Degree of hydration*

The hydration percentage of the gels was measured at 37 °C using aqueous media at different pH (2, 7.4 and 10), each measurement made in triplicate. The percentage of the degree of hydration of the gels was calculated using the following formula: $\%H_e= [(W_e-W_o)/W_o] \times 100$, wherein He is the degree of hydration at equilibrium, $W_e$ is the weight of the cross linked gel at equilibrium and $W_o$ is the initial weight of the gel. The degree of hydration at equilibrium is depicted in figure 4. It is observed that as the concentration of crosslinking agent increases, the maximum hydration reached at equilibrium decreases. This is because the hydrogel has a higher degree of crosslinking and therefore is more compact and the solvent penetrates less in the polymer structure. The degree of hydration at equilibrium of the gels cross linked with genipin ranges from 1200% for the hydrogels prepared with 5% crosslinker and 3000% for those prepared with only 0.5%.

It was also observed that at pH= 7.4 the degradation of the hydrogel cross linked with 1% genipin started at 48 hours, however at pH= 2 and pH= 10 the degradation of the hydrogel started from the sixth and seventh day respectively. At pH= 7.4 the hydrogels reach a higher degree of hydration. This is because at pH= 2 the uncrosslinked amino groups of the gelatin are protonated (pKa= 4.8) and therefore they will interact ionically with the carbonyl groups present in the polymer matrix, resulting in ionic interactions that provide a higher degree of crosslinking of the structure of the hydrogel. At pH 10 the carbonyl groups (pKa = 9.4) are negatively charged and can interact with the free amino groups present in the structure. However, at pH = 7.4 the ionic groups are partially charged and the ionic interaction among them has lower strength, this allows greater penetration of the water molecules in the matrix, which entails a higher degree of hydration.

*Mechanical stability.*

1. Time scans

Deformation, time and frequency dynamic mechanical tests were carried out to determine the storage and losses modules of each cross linked system and estimate the gel point of each system. The time scans were carried out setting a 2% deformation and 0.5 Hz at 25 °C.

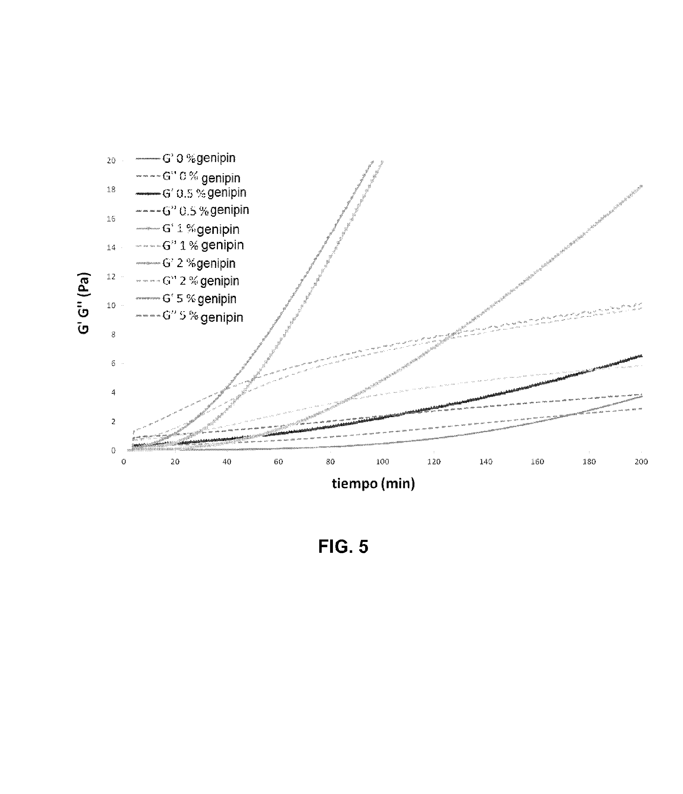

Figure 5 shows the transition of the gelatin-sodium hyaluronate hydrogels cross linked with different concentrations of genipin, from their state in solution to a viscoelastic solid (gel state). This transition occurs in the course of the reaction, which allows to study the evolution of the properties of the material during the kinetics of the crosslinking reaction. The increase of the storage modulus (G') as a function of the time is observed, indicating the increase of the solid nature of the material.

2. Frequency scans.

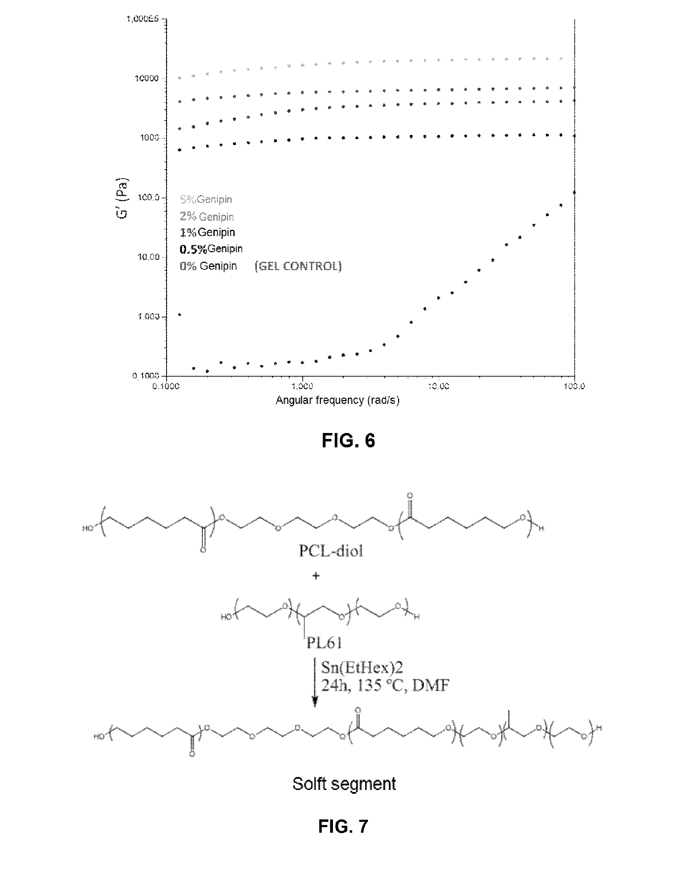

The frequency scan was carried out with 2% deformation between 0.01 and 100 Hz at 25 °C. The stabilization of G' towards the final stages (Fig. 6) defines the viscoelastic properties of the gel formed.

It is observed how when increasing the concentration of crosslinker, the value of the storage modulus and the loss modulus increases (Table 1), indicating increased stability of the gel. In the control gel (the one that does not contain the crosslinking agent) the linear stability in the value of both modules (G' and G") was not reached, indicating that the gel structure is not stable.

*Table 1. Values of the storage (G') and loss (G") moduli in gel state*

|  | Control Gel (0% Genipin) | Genipin 0.5% | Genipin 1% | Genipin 2% | Genipin 5% |
|---|---|---|---|---|---|
| G' | 378 | 1060 | 3740 | 6400 | 20120 |

(continued)

|  | Control Gel (0% Genipin) | Genipin 0.5% | Genipin 1% | Genipin 2% | Genipin 5% |
|---|---|---|---|---|---|
| (Pa) |  |  |  |  |  |
| G" (Pa) | 61 | 94 | 212 | 385 | 1300 |

*Thermal Stability*

Thermogravimetric analyses (TGA) of the systems cross linked with genipin were carried out and were compared with the uncrosslinked gel (Control HG). When the concentration of the crosslinker is increased, the maximum degradation temperature as well as the temperature at which 10% of the system has been degraded increase due to a greater crosslinking and stability of the gel (Table 2).

*Table 2. Degradation temperatures of the gelatin-sodium hyaluronate system cross linked with different concentrations of genipin*

| SAMPLE | $T_{10\%}(°C)^1$ | $T_{max}(°C)^2$ |
|---|---|---|
| CONTROLHG | 105 | 312 |
| Genip. HG 0.5% | 105 | 314 |
| Genip. HG 1% | 110 | 317 |
| Genip. HG 2% | 135 | 319 |
| Genip. HG 5% | 140 | 322 |
| 1) $T_{10\%}$ = Temperature at which 10% of the system has been degraded. 2) $T_{max}$= Temperature at which the degradation rate is maximum, according to the derivative of the curve of the thermogram. | | |

### Example 2. Polyurethane film

<u>Materials</u>

The reagents and solvents listed below were purchased from Sigma-Aldrich and used directly without prior purification: poly(tetramethylene glycol ether) (PTMG) alcohols number 55.1, poly(caprolactone diol) (PCL-diol) with molecular weight of 2000 Da, pluronic-L61 (PL61) with molecular weight of 2000 Da, tetrahydrofuran (THF), anhydrous *N,N*-dimethylformamide (DMF), tin 2-ethylhexanoate and methyl L-lysine diisocyanate.

<u>Methods</u>

*Synthesis of the chain extender*

The chain extender was prepared from PCL-diol and PL61 at molar ratio (1:1) according to the following experimental procedure.
A solution of PCL-diol was prepared (6.62 g, 3.3 mmol) in anhydrous DMF (50 ml) under nitrogen atmosphere, it was heated 20 min at 135 °C. Next tin 2-ethylhexanoate (II) (30 μl, 0.093 mmol) and pluronic-L61 (7.04 g, 6.96 ml, 3.5 mmol) were added. The reaction was stirred 24 hrs at 135 °C obtaining in this manner the chain extender (50PCL-diol-50PL61) (Fig. 7).

*Synthesis of the polyurethane*

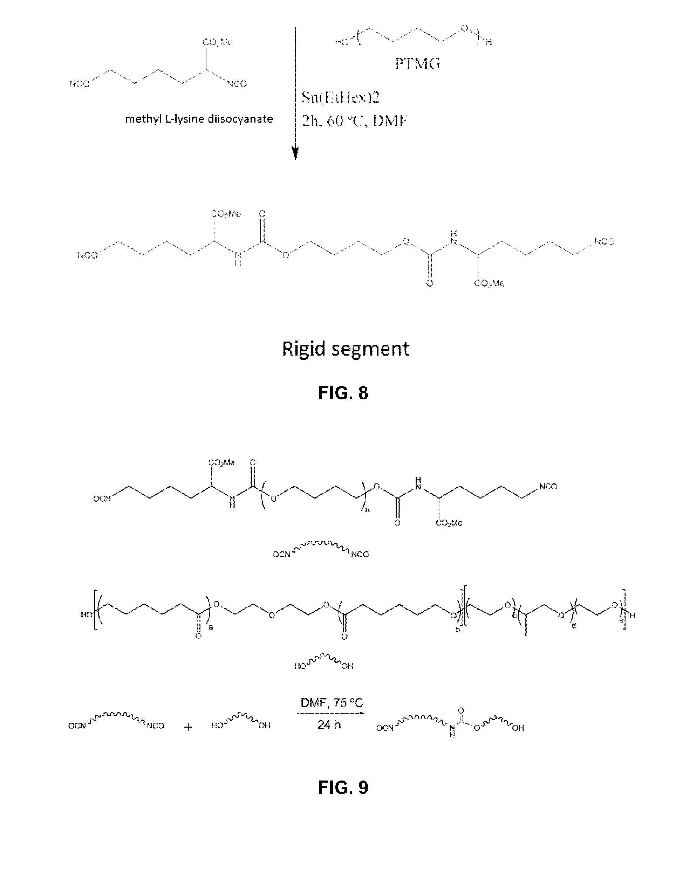

First, the pre-polymer was obtained by adding a slight excess of methyl L-lysine diisocyanate (2.38 g, 2.23 ml, 11.2 mmol) with PTMG (7.62 g, 3.7 mmol) in anhydrous DMF (60 ml) and tin 2-ethylhexanoate (II) (30 μl, 0.093 mmol) was added. The mixture was stirred 2 hrs at 60 °C to obtain the rigid segment (Fig. 8). Secondly, the chain extender was added to the resulting pre-polymer to obtain the segmented polyurethane (Fig. 9). The reaction was carried out under nitrogen atmosphere at 75 °C for 24 hrs.

The resulting polyurethane was precipitated in water. Finally, it was dried under vacuum at 50 °C for 48 hrs and was designated SPU321-LDI- [50PCL-diol-50PL61].

Characterization

The products were characterized by means of spectroscopic techniques of Proton Nuclear Magnetic Resonance ($^1$H-RMN) and Attenuated Total Reflection Fourier Transform Infrared (ATR-FTIR). The resonance spectra were recorded on Varian Inova-400 MHz equipment, using deuterated chloroform ($CDCl_3$) as solvent. The infrared spectra were obtained in Perkin-Elmer Spectrum One equipment, performing a scan of 4000 to 600 cm$^{-1}$. 32 scans were carried out with a resolution of 4 cm$^{-1}$.

The number average molecular weight ($M_n$), weight average molecular weight ($M_w$) and the polydispersity index (PDI) were determined by means of size exclusion chromatography (SEC) using a Perkin Elmer chromatograph equipped with an isocratic pump Series 250 and a refractive index detector Series 200. The samples were eluted through three polystyrene-divinylbenzene PL-gel columns connected in series of 500, $10^4$ and $10^5$ nm pore size (Polymer Laboratories) thermostatized at 70 °C. N,N-dimethyl formamide (DMF) with 0.1% LiBr at a flow of 0.3 ml/min was used as mobile phase. For the calibration, polystyrene standards with molecular weight of between 10300 and 480000 Da were used.

By using Differential Scan Calorimetry (DSC) the melting temperature as well as the increase of the enthalpy associated with the process was determined using a calorimeter (Perkin Elmer DSC8500) previously calibrated with Indium and Zinc standards. For this, the dried sample of the different materials was deposited inside aluminium capsules which were sealed and heated at constant rate of 5 °C/min, with a N2 flow of 20 ml/min, in the range of temperatures from -20 to 100 °C. The temperatures obtained from the thermogram corresponding to the second scan were taken.

The thermogravimetric analysis (TGA) was carried out in TGA Q500 (TA) equipment between 25 and 600 °C under nitrogen atmosphere at 10 °C/min.

The dynamic mechanical analysis was carried out in a differential mechanical analyzer METTLER DMA 861e, carrying out temperature scans between 0 °C and 80 °C at different frequencies (0.5, 1, 3, 5 and 10 Hz).

Results and discussion

The characteristics of the segmented polyurethanes can be modified by simply changing the ratio of the hard and soft segments; that is, by varying the molar ratios between the diisocyanate, the polydiol and the chain extender.

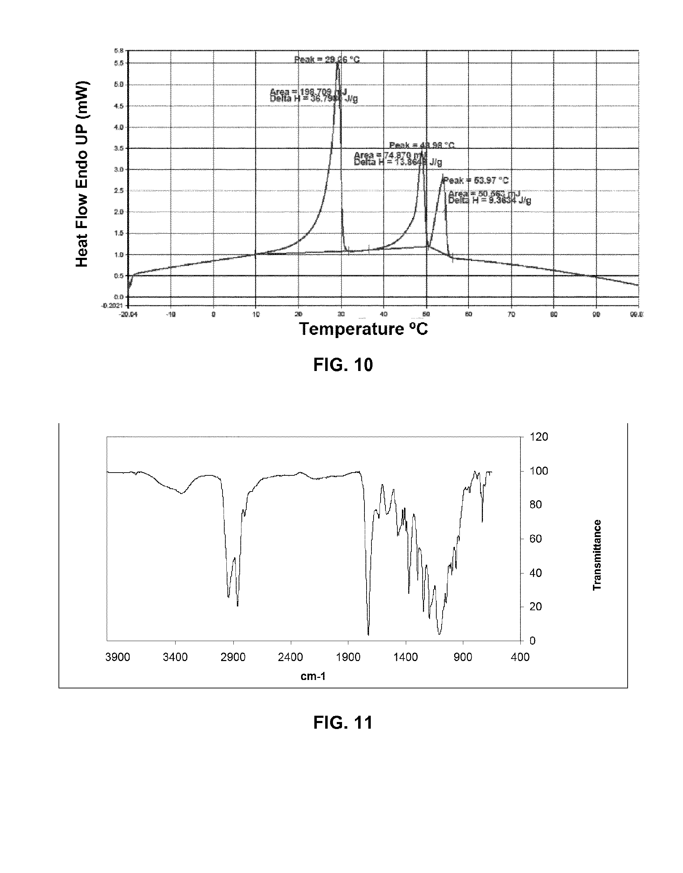

The polyurethane obtained was characterized by DSC (Fig. 10). Three main endothermic transitions were observed (29, 49 and 54 °C). These transitions are attributed to the melting of the crystalline domains formed by the polyurethane chains. The first corresponds to the rigid segment formed by PTMG and the last two to the soft segment formed by PCL-diol. The occurrence of two melting enthalpy transitions relative to the PCL-diol is due to the existence of two different crystalline domains, one with higher degree of orientation in space ($T_f$ = 54 °C) and one with lower degree of orientation ($T_f$ = 49 °C).

The characterization by ATR-FTIR (Fig. 11) of the polyurethane showed the corresponding characteristic bands: $V_{NH}$ = 3346 cm$^{-1}$, $V_{CH2}$ = 2935 and 2859 cm$^{-1}$, $V_{co}$ = 1723 cm$^{-1}$; $V_{CN}$ = 1549, 1243 cm$^{-1}$, $V_{coc}$ = 1190 cm$^{-1}$; $v_{c-c}$ = 1046 cm$^{-1}$. The absence of absorption band at 2273 cm$^{-1}$, characteristic of the isocyanate group, indicates that all the diisocyanate reacted and the possible excess was removed by dialysis.

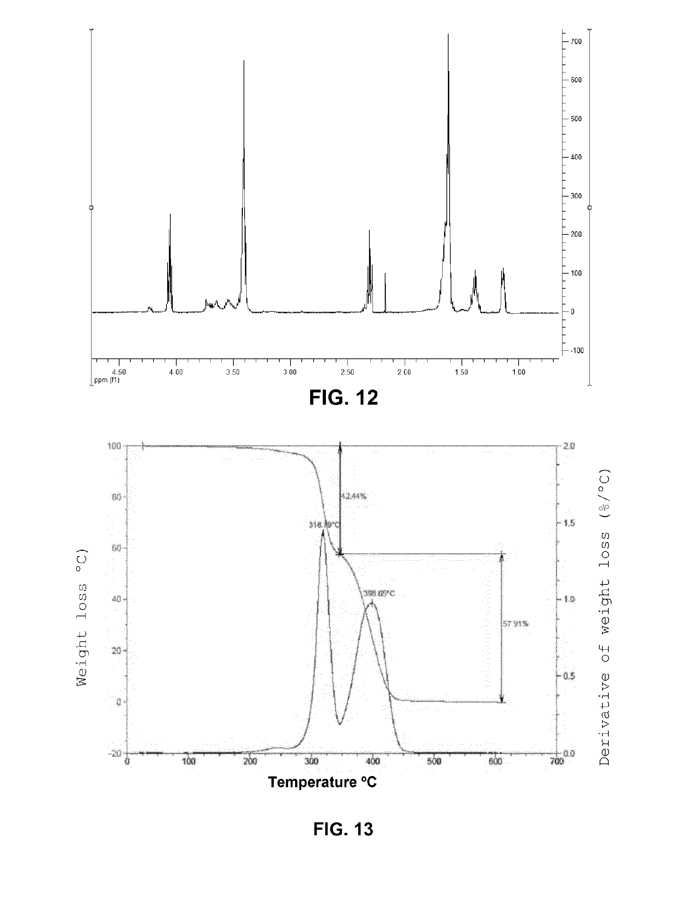

The $^1$H-RMN spectrum (Fig. 12) shows signals characteristic of each of the components of the polyurethane. Thus, at 2.25 ppm appears the signal of the methylene group (-CH$_2$-) in position $\alpha$ to the carboxylate of the PCL-diol, at 1.05 ppm appears the methyl group (-CH$_3$) of the PL61, the signals corresponding to PTMG appear at 3.4 ppm (-CH$_2$-O-) and at 1.6 ppm (-CH$_2$-C-) overlapped together with signals of the other two components. And the characteristic signals of the LDI appear at 3.6 ppm and 4.1 ppm corresponding to methyl (-CH$_3$) and the CH group in position $\alpha$ to the ester, respectively.

The thermogravimetric analysis (Fig. 13) of the SPU321-LDI-[50PCL- diol-50PL61] shows two weight losses attributable to the thermal degradation of the two segments of polyurethane. This indicates that the polyurethane has two distinct segments, one rigid and one soft, the maximum of which according to the derivative of the degradation curve are 318 and 398 °C respectively. Based on the TGA measures it can be said that the SPU321-LDI-[50PCL-diol-50PL61] has a content of 42% of the rigid segment and 58% of the soft segment.

The molecular weight and polydispersity distribution obtained by SEC, as well as the loss and storage modulus and the value of Tg obtained by DMTA for the SPU321-LDI-[50PCL-diol-50PL61] is shown in table 3.

*Table 3. Characterization by SEC and DMTA of the SPU321-LDI-[50PCL-diol-50PL61].*

| $M_w$ (Da) | $M_n$ (Da) | PDI | G' (MPa) | G" (MPa) | $T_g$ (°C) |
|---|---|---|---|---|---|
| 19109 | 6983 | 2.73 | 500 | 2000 | 22 |

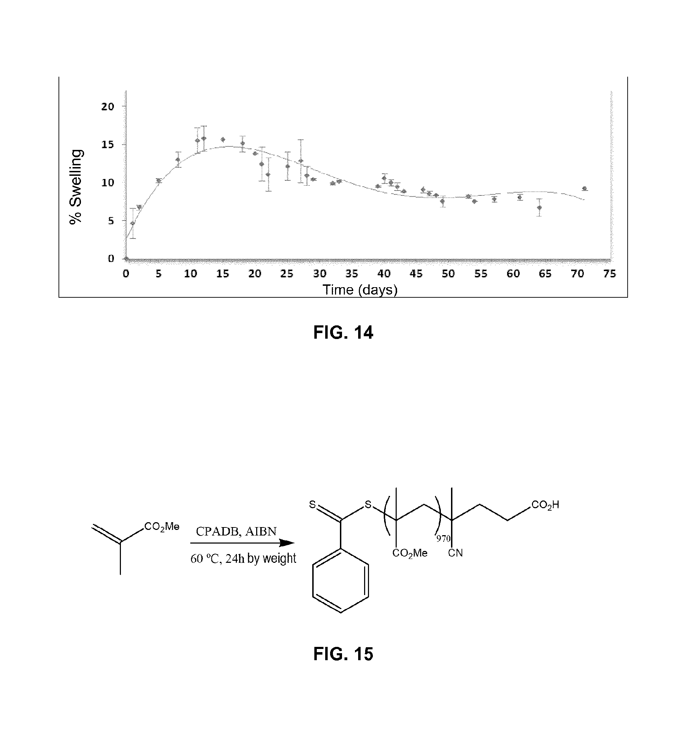

The hydration percentage of the SPU321-LDI-[50PCL-diol-50PL61] was measured at 37 °C in distilled water, each measurement made in triplicate. The percentage of the degree of hydration of the polyurethane was calculated using the following formula: $\%H_t = [(W_t - W_o)/W_o] \times 100$, wherein $H_t$ is the hydration percentage at time t, $W_t$ is the weight of the polyurethane at time t and $W_o$ is the initial weight of the polyurethane. The hydration profile showed a maximum value of 10 to 15% at 15 days (Fig. 14) and then declined to reach a stable value of 9% as of day 40. This behaviour is due to a viscoelastic response of the polyurethane film. In the beginning the system is in solid state, forming a rigid crystal lattice (dry film). When the system begins to hydrate, the chains forming the amorphous domains swell and become more flexible reaching the maximum degree of hydration. However, with time the system tends to a state of minimum energy, so that the chains begin to relax and contract, expelling water molecules that had penetrated in the polymer network, until reaching a stable degree of hydration. This behaviour is characteristic of viscoelastic gels.

***Example** 3. **Synthesis** of block copolymers*

<u>Materials</u>

The [2-(methacryloyloxy)ethyl]trimethyl ammonio (aqueous solution 80% w) (MAETMA) and the 4,4'-azobis (4-cyano) valeric acid (V501) were purchased at Sigma-Aldrich. The low molecular weight heparin (bemiparin, 111.3 IU/mg) was donated by ROVI pharmaceutical laboratories. The methyl methacrylate (MMA) was obtained from Acros Organics, the 2,2'-azobisisobutyronitrile (AIBN) was obtained from Merck and it was recrystallized in ethanol before use. The pro-adrenomedullin N-20 (PAMP) with molecular weight of 2460 Da was obtained from Phoenix Pharmaceuticals INC.

<u>Methods</u>

The block copolymers were synthesized by controlled radical polymerization, through the mechanism of Reversible Addition-Fragmentation Chain Transfer (RAFT). For this a RAFT transfer agent (4-cyanopentanoic acid dithiobenzoate, CPADB) previously synthesized and characterized was used.

First, the hydrophobic block was synthesized by mass polymerization from CPADB (28 mg, 0.1 mmol) and methyl methacrylate (MMA) (10.6 ml, 100 mmol), using AIBN (1.64 mg, 0.01 mmol) as radical initiator. A stream of nitrogen was passed through this mixture for 30 min and it was heated at 60 °C for 24 hrs (Fig. 15).

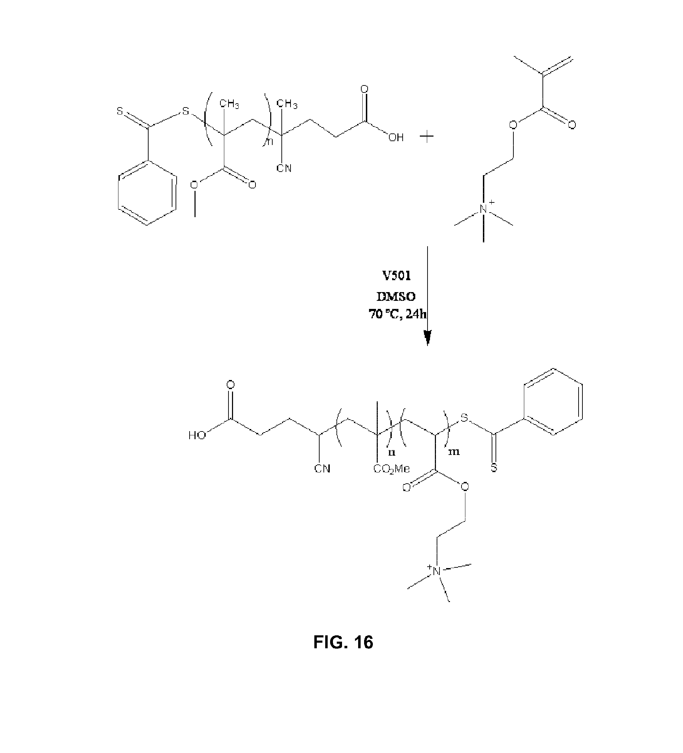

From the RAFT macroinitiator of PMMA, the polymerization of the second block was carried out using [(2-methacryloyloxy)ethyl] trimethyl ammonium chloride (MAETMA). For this, in a 250 ml flask 6.2 ml of a PMMA 0.6 mM solution were dissolved in DMSO (3.74 mmol), 1.8 ml of a 0.42 mM 4,4'-azobis (4-cyano) valeric acid (0.75 mmol) solution in DMSO and various amounts of MAETMA (374, 187 and 93.5 mmol respectively) in 34 ml of DMSO to obtain three block copolymers with different chain length of the hydrophilic component (PMAETMA) (Fig. 16).

A stream of nitrogen was passed for 30 min and it was heated at 70 °C for 6 hrs. Finally, the resulting product was dialyzed in water for 3 days using tubular dialysis membranes with a 3.5 kDa pore size.

<u>Characterization</u>

The products were characterized by ATR-FTIR and $^1$H-RMN, using DMSO-d6. The number ($M_n$) and weight ($M_w$) average molecular weights, as well as the polydispersity (PDI) were determined by SEC at 70 °C, using DMF as mobile phase with 0.1% LiBr at a flow of 0.3 ml/min. For the calibration, poly(methyl methacrylate) standards with molecular weight of between 2.9 and 480 kDa obtained from Polymer Laboratories were used.

For the determination of the glass transition temperature a Perkin Elmer DSC7 apparatus was used. The DSC study was carried out by depositing the dried sample of the different materials in the interior of aluminium capsules that were sealed and heated at constant speed of 10 °C/min, with a $N_2$ flow of 20 ml/min, with temperatures in the range of -20 to 180 °C. The temperatures obtained from the thermogram corresponding to the second scan were taken.

The thermogravimetric analysis (TGA) was carried out under $N_2$ atmosphere in TGA Q500 (TA) equipment between 25 and 600 °C with a heating rate of 10 °C/min.

<u>Results and discussion</u>

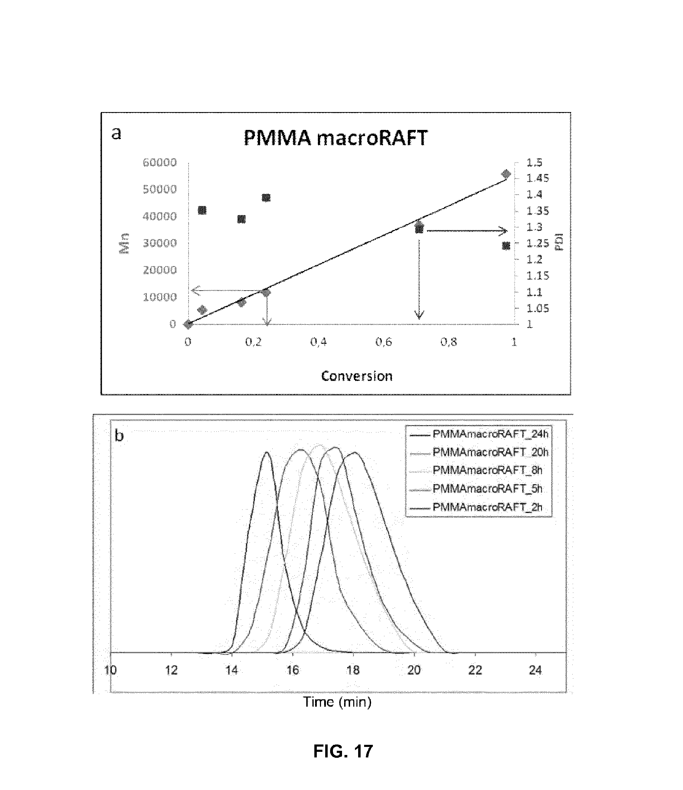

The SEC results (Fig. 17a) showed that the molecular weight increased linearly with the conversion, showing that the polymerization was carried out under controlled conditions with a polydispersity below 1.3. This was also demonstrated by the chromatograms of molecular weight distribution obtained versus the reaction time where a unimodal distribution at all times was obtained (Fig. 17b).

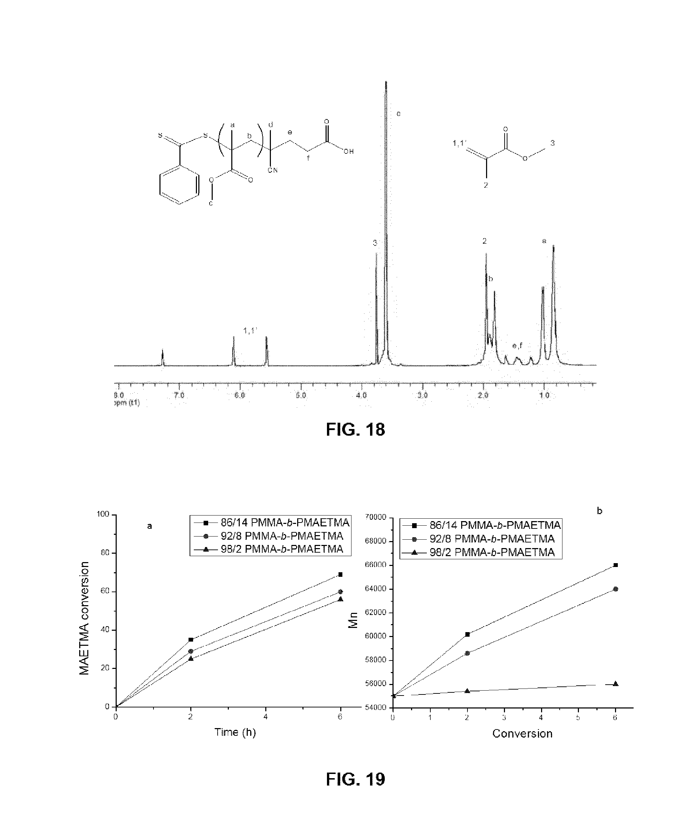

Figure 18 shows the $^1$H-RMN spectrum of the PMMA macro RAFT obtained without prior purification. From the integration ratio of the signals corresponding to the residual monomer (5.6 and 6.1 ppm) and those corresponding to the PMMA product (0.8-1.2 ppm) a final conversion of 97% was calculated. This value is also corroborated with a gravimetric study.

From the macroinitiator obtained (PMMA macro RAFT), different block copolymers were prepared, starting from blocks of the same molecular weight of

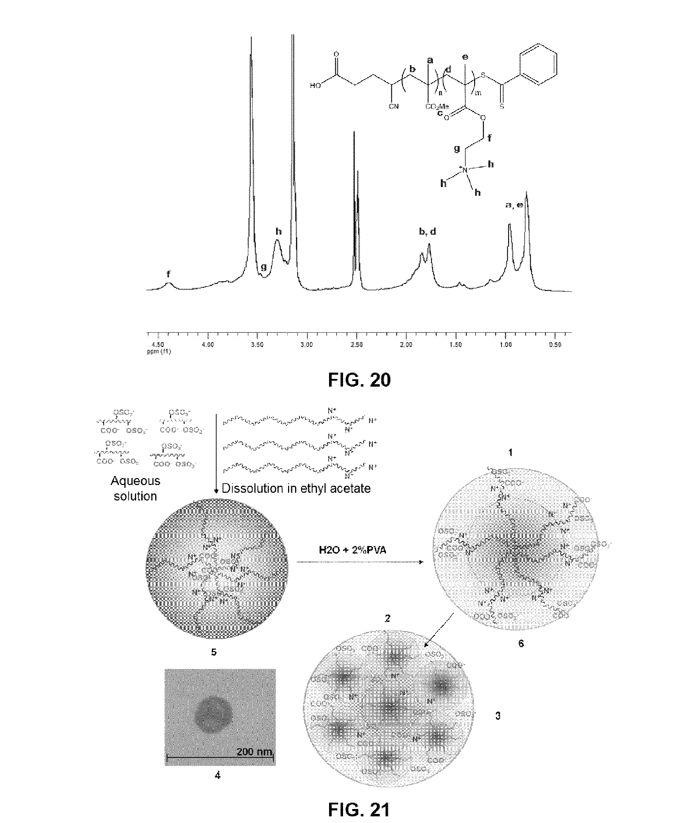

PMMA and varying the amount of PMAETMA to thereby control the degree of hydrophilicity of the system (Table 4). Good control of the polymerization was obtained in all the systems, resulting in polydispersities lower than 1.5 (Table 4). The evolution of the conversion with time and of the distribution of the molecular weight with the conversion is shown in figure 16. At low conversions, the molecular weight increased linearly with the conversion, however, at conversions higher than 60% the kinetic stops being of first order, probably due to the presence of transfer reactions that decrease the generation of radicals able to maintain the process of propagation of the polymerization. The polydispersity in all the systems was less than 1.5 (Fig. 19). The copolymers were characterised by $^{1}$H-RMN (Fig. 20). For the calculation of the molar ratio of both blocks in the different copolymeric systems the signals between 4.2 and 4.4 ppm corresponding to the PMAETMA and the signals between 0.3 and 1.2 ppm corresponding to the methyl groups of the two blocks were analyzed. The molar composition of both blocks in the copolymer was calculated from the value of the integrals (Table 4).

*Table 4. Ratio of the RAFT macroinitiator (PMMA) and the MAETMA monomer in the feed and in the resulting copolymer. Molecular weights obtained from the experimental values of SEC. The conversion was calculated by $^{1}$H-RMN*

| $F_{PMMA/MAETMA}$ (Feed) | $F_{PMMA/MAETMA}$ (Copolymer) (calculated by $^{1}$H-RMN) | (n) PMMA units | (m) PMAETMA units | $Mn_{teo}$ (Da) | $Mn_{exp}$ (calculate by SEC) (Da) | PDI | % conversion at 6 hrs |
|---|---|---|---|---|---|---|---|
| 90/10 | 86/14 | 970 | 158 | 69000 | 66000 | 1.40 | 67 |
| 95/5 | 92/8 | 970 | 84 | 61200 | 64000 | 1.41 | 60 |
| 97.5/2.5 | 98/2 | 970 | 20 | 57900 | 56000 | 1.45 | 56 |

### Example 4. Preparation of nanoparticles loaded with bemiparin

Materials

Poly(vinyl alcohol) (PVA) ($M_{w}$= 31000-50000 g/mol, 98-99% hydrolyzed) was obtained from Sigma-Aldrich. Chitosan (PROTASAN UP CL213, $M_{w}$= 150000-400000 g/mol, 75-90% deacetylation degree) was obtained from Novamatrix. Eudragit RS PO ($M_{w}$= 150000 g/mol, 4.48-6.77% ammonio methacrylate units) was obtained from Degussa. PLGA (RESOMER RG 504, molar ratio 50:50, $M_{w}$ = 40000 g/mol) was obtained from Boehringer Ingelheim Pharma GmbH & Co. The bemiparin was donated by ROVI pharmaceutical laboratories. The block copolymers p(MMA$_{n}$-b-MAETMA$_{m}$) were synthesized by controlled radical polymerization, RAFT.

Methods

Different formulations of nanoparticles loaded with bemiparin have been prepared. Different techniques have been used for the preparation of these systems: on the one hand the method of multiple emulsion or triple emulsion using different polymer phases and on the other, nanoparticles have been prepared based on the formation of polyelectrolyte complexes from bemiparin and chitosan.

*Nanoparticles prepared by multiple emulsion.*

The preparation of these systems is based on an aqueous solution of bemiparin (20 mg/ml). 100 ml of a solution of polymer (10 mg/ml) in ethyl acetate are added drop wise over 10 ml of this aqueous phase and the system is kept under constant stirring by ultrasound probe (40 A). Thus a first emulsion is formed which is again added drop wise over 400 ml of an aqueous solution of PVA (20 mg/ml) which acts as stabilizing agent. The system is kept again under stirring by ultrasound (40 A). After washing and centrifugation at 12,000 rpm to remove the stabilizer, the particles are isolated by lyophilisation. The bemiparin load of these systems was between 10% and 15% by weight with respect to the concentration of polymers (Fig. 21).

*Nanoparticles prepared from bemiparin and chitosan.*

The preparation of these systems is based on the formation of polyelectrolyte complexes of bemiparin with chitosan starting from aqueous solutions of both components at pH = 5 previously adjusted with a solution of acetic acid (1% v/v). The method for the preparation involves the drop wise addition of the solution of bemiparin (0.03% w/v, 5 ml, pH = 5) over the chitosan solution (0.1% w/v, 1.5 ml, pH = 5) keeping the mixture under stirring with a homogenizer at 3400 rpm for 15 minutes. The isolation of the nanoparticles is carried out by centrifugation at 12,000 rpm for one hour and subsequent lyophilisation. The bemiparin load of this system was 45% by weight with respect to the concentration of chitosan.

Characterization of the systems

The products were characterized by ATR-FTIR (4000 to 600 cm-1), TGA (25 to 600 °C under nitrogen atmosphere at 10 °C/min) and DSC (the scan was carried out at a constant rate of 10 °C/min, with a $N_2$ flow of 20 ml/min, with temperatures in the range of -20 to 180 °C) to determine the glass transition temperature of the NP. For the calculation of the $T_g$, the temperature at which half of the difference in specific heat between the vitreous amorphous and elastomer amorphous states is reached is taken, that is, $Tg= \frac{1}{2} \Delta C_p$. This point corresponds to the inflection point of the change in slope in the thermogram of the second scan.

The external and internal morphology of the nanoparticles was analyzed by scan electron microscopy (SEM) using Philips XL 30 ESEM equipment at a voltage of 15 keV. The samples were prepared by adding one drop of an aqueous solution (0.01 mg/ml) of the same on a glass slide of 13 mm diameter and 1 mm thick. The solvent was allowed to evaporate at room temperature and the sample was metallised using chromium coating.

The microanalysis of the surface of the nanoparticles was carried out by energy dispersive X-ray (EDAX) at 15 keV.

The measurements of particle size and Potential Zeta (PZ) were carried out using the technique of dynamic light scattering (DLS) using Zetasizer Model Nano ZS equipment (Malvern Instruments, UK) equipped with a 633 nm red laser (He/Ne). In order to perform the measurements 0.01 mg/ml solutions were used, the cell was equilibrated at 25 °C for 5 minutes and 5 replicates of each measurement of 20 scans were made. The PZ was calculated using the Henry equation (Equation I). From this equation the PZ can be calculated by a simple measurement of electrophoretic mobility. When working in aqueous solutions and with low concentrations of electrolyte, the Smoluchowski approximation can be applied which assumes that the Henry function has a value of 3/2, whereby the PZ can be calculated as:

$$U_E = 2\varepsilon_{zf(ka)} / 3\eta \rightarrow z \approx U_E\, \eta/\varepsilon \qquad \text{(Equation I)}$$

Wherein $U_E$= electrophoretic mobility, z= potential zeta (PZ), $\varepsilon$= dielectric constant, $\eta$= viscosity, and f(ka)= Henry function.

The measurements of biological activity of the free and encapsulated bemiparin were carried out by cell proliferation assay. For this, BaF32 cells were used, which are a type of lymphocyte B, the cell signalling of which is mediated by the formation of a complex between the bemiparin, the FGF-2 and its corresponding FGFR1c receptor on the cell surface.

Results and discussion

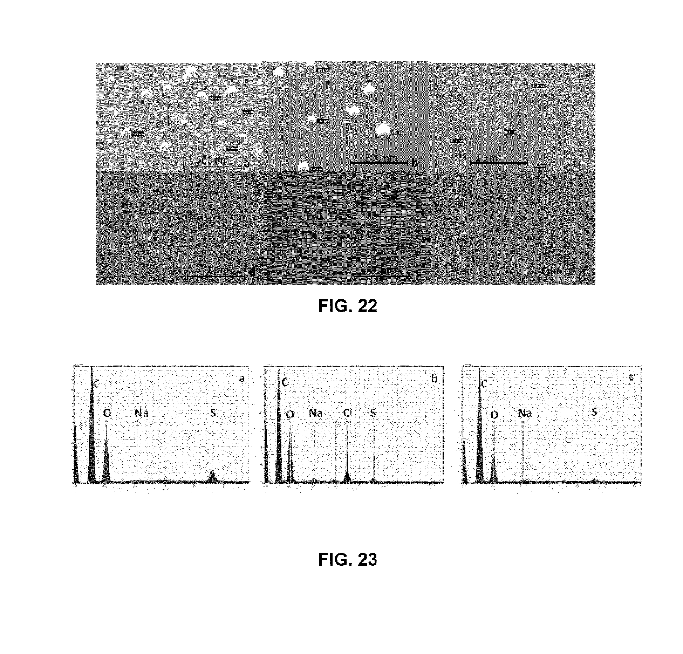

Particles with a spherical morphology and a particle size in the nanometre range (Fig. 22) were obtained in all cases. The elemental analysis of the surface of the nanoparticles was carried out using the technique of energy dispersive X-ray spectroscopy (EDAX). The sulphur signal corresponding to the presence of bemiparin was observed in all the systems (Fig. 23).

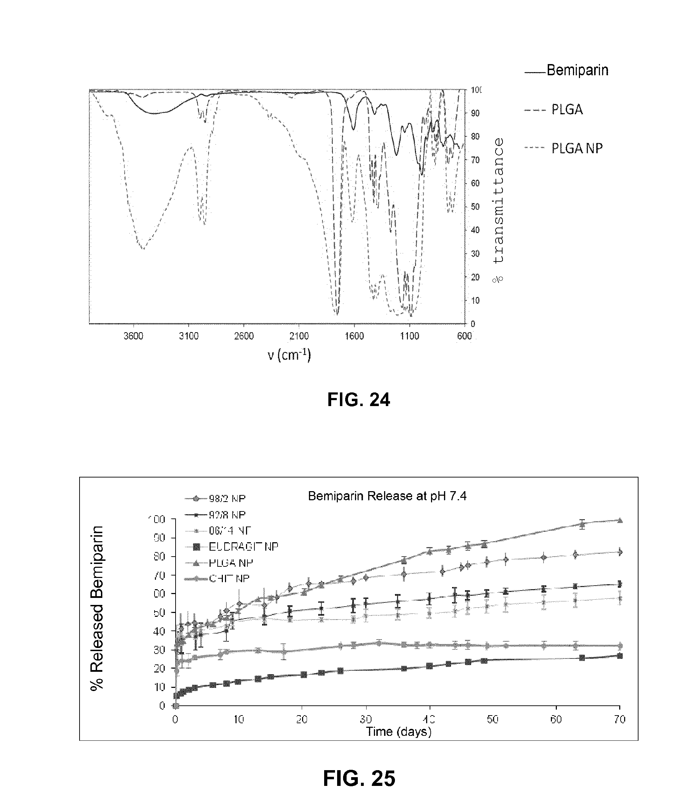

The characterization by means of infrared spectroscopy (FTIR) allowed to observe in the nanoparticulate system the signals characteristic of both the polymer ($V_{co}$ = 1700 cm-1) and the bemiparin ($V_{co}$ = 1600 cm-1, $V_{OH}$ = 3500 cm-1) (Fig. 24).

The macroinitiator RAFT (PMMA) had an experimental Tg of 117 °C. The RAFT block copolymers have a single glass transition mainly corresponding to the PMMA block. The block corresponding to PMAETMA does not have its own glass transition because it does not equimolarly coexist but it is at a very low ratio to the PMMA segment. It can be said that the PMAETMA segment does not reach the critical concentration for phase segregation to exist between the various blocks, but it does affect the Tg value corresponding to the block of PMMA. It is observed that when increasing the chain length of the PMAETMA, the Tg decreases slightly due to an increase of the hydrophilic component in the system, having the same effect as a plasticizer, which leads to the decrease of the Tg.

When preparing the nanoparticles of these block copolymers, the self-association of the hydrophobic block and the hydrophilic block occurs, which is enriched with the aggregation of the bemiparin in the system. Therefore, the phase segregation between the different blocks is favoured, and two glass transition values can be observed, corresponding to each of the blocks ($T_{gi}$ corresponds to the value of PMAETMA, $T_{g2}$ corresponds to the value of PMMA) (Table 5).

*Table 5. Thermogravimetric characterization of the various copolymeric systems and their corresponding nanoparticle systems, by TGA and DSC*

| SAMPLE | $T_{10\%}(°C)$[3] | $T_{max}(°C)$[4] | $T_{g1}(°C)$ | $T_{g2}(°C)$ |
|---|---|---|---|---|
| BEMIPARIN | 120 | 242 | - | - |
| PLGA | 270 | 307 | 46.9 | - |
| PLGA NP loaded with BEMIPARIN | 265 | 321 | 47.6 | - |
| EUDRAGIT | 330 | 370 | 68.0 | - |
| EUDRAGIT NP loaded with BEMIPARIN | 355 | 403 | 44.9 | 89.2 |
| 86/14 copolymer | 220 | 384 | - | 86 |
| 86/14 NP loaded with BEMIPARIN | 267 | 386 | 39 | 112 |
| 92/8 copolymer | 240 | 386 | - | 93 |
| 92/8 NP loaded with BEMIPARIN | 275 | 396 | 36 | 114 |
| 98/2 copolymer | 245 | 394 | - | 110 |
| 98/2 NP loaded with BEMIPARIN | 295 | 405 | 38 | 118 |
| CHITOSAN | 194 | 229 | - | - |
| CHITOSAN NP loaded with BEMIPARIN | 135 | 246 | | |
| 3) $T_{10\%}$= Temperature at which 10% of the system has been degraded. 4) $T_{max}$= Temperature at which the degradation rate is maximum, according to the derivative of the curve of the thermogram. | | | | |

The mean hydrodynamic diameter and the values of PZ of the nanoparticles are shown in Table 6. The nanoparticles without bemiparin had an average size between 115 and 290 nm. It was observed that the particles prepared from the RAFT copolymers have a greater size due to the positive charge they have on the polymer chains. This charge means that there are forces of electrostatic repulsion, causing an increase in size in the nanoparticles.

When the nanoparticles are loaded with bemiparin, the size decreases significantly in the systems prepared with the RAFT copolymers due to neutralization of positive charges of the copolymer that are offset by the negative charges of the bemiparin.

In the PLGA system, no significant differences were observed in the average size of the nanoparticles because this copolymer does not have charge on its surface.

The value of PZ observed (Table 6) in the nanoparticles prepared with RAFT copolymers, EUDRAGIT and CHITOSAN without bemiparin was a positive value (27- 42 mV) due to the presence of the charge of the quaternary ammonium or of the amino group of the chitosan. In the case of the PLGA nanoparticles without bemiparin a small negative surface charge was observed possibly due to the charge of the groups ($OH^-$, $COO^-$ of the chain ends). When the bemiparin is incorporated in all the systems of nanoparticles, the PZ becomes highly negative, ranging between (-10 and -50) mV. This shows that the bemiparin has joined the system and tends to be on the surface of the nanoparticle together with the more hydrophilic component of the polymer system (Fig. 5). Some decrease in absolute value of the PZ is observed with the increase of the chain length of the MAETMA block in the copolymers. This can be explained due to the presence of a greater number of positive charges that are neutralized by the negative charges present in the bemiparin, so that the value of the PZ on the surface of the nanoparticle is lower because there are fewer charged groups.

*Table 6. Measurements of the hydrodynamic diameter and of the PZ of the nanoparticles from DLS.*

| SAMPLE | MEAN DIAMETER (nm) | PZ (mV) |
|---|---|---|
| PLGA NP loaded with bemiparin | 119 ± 11 | -49 ± 4 |
| EUDRAGIT NP loaded with bemiparin | 135 ± 4 | -38 ± 4 |
| 86/14 NP loaded with bemiparin | 216 ± 4 | -9 ± 1 |
| 92/8 NP loaded with bemiparin | 195 ± 9 | -11 ± 2 |
| 98/2 NP loaded with bemiparin | 174 ± 2 | -23 ± 1 |
| CHITOSAN loaded with bemiparin | 99 ± 12 | -22 ± 4 |
| 86/14 NP | 143 ± 24 | 37 ± 1 |
| 92/8 NP | 183 ± 41 | 36 ± 1 |
| 98/2 NP | 286 ± 22 | 27 ± 1 |
| EUDRAGIT NP | 236 ± 98 | 36 ± 5 |
| PLGA NP | 114 ± 8 | -4 ± 2 |
| CHITOSAN NP | 200 ± 55 | 42 ± 12 |

The calculation of the drug encapsulation efficiency (EE) was conducted by an indirect method, from the analysis by high resolution liquid chromatography (HPLC) of the unencapsulated bemiparin present in the supernatant obtained after isolation by centrifugation of the nanoparticles. All the systems prepared had encapsulation percentages near 100% (Table 7).

*Table 7. Measurements of the EE of the bemiparin in the various systems of nanoparticles measured by HPLC coupled to a UV-Vis detector.*

| NP SYSTEM | %EE |
|---|---|
| PLGA NP | 89 ± 3 |
| EUDRAGIT RS PO NP | 94 ± 2 |
| 86/14 NP | 98 ± 2 |
| 92/8 NP | 96 ± 3 |
| 98/2 NP | 95 ± 4 |
| CHITOSAN NP | 99 ± 1 |

The study of bemiparin release was carried out using HPLC with UV-Vis detector, setting the wavelength at 242 nm. The mobile phase used was Milli-Q water adjusted with glacial acetic acid at pH = 3, 1 ml/min.

The release of bemiparin occurs primarily by diffusion of the drug through the swollen polymer matrix. The more hydrophilic the polymer system is, the greater is the amount of solvent molecules that penetrate into the nanoparticle and, therefore, better diffusion and increased release of the same into the medium. However, in the case of the CHITOSAN, EUDRAGIT and the RAFT block copolymers, there is a positive charge due to the amino group or the quaternary ammonium salt of the choline forming them, respectively. This positive charge interacts through the formation of ionic bonds with the sulfonic and carboxylic groups present in the bemiparin, whereby the chemical composition of the polymer system will affect the release of bemiparin into the medium. At pH = 7.4 it was observed that the release of bemiparin is higher the lower the content of the MAETMA block is due to lower positive charge density and, therefore, to a lower ionic retention of the bemiparin. In the case of EUDRAGIT, it is a statistical terpolymer in which the distribution of the MAETMA chain is not forming a particular block, but is randomly distributed along of the polymer chain, so that the system is more hydrophobic and there is more interaction with the molecules of bemiparin. This leads to greater retention of the drug and less diffusion into the medium, whereby the release of bemiparin in the EUDRAGIT system was slower than in the other systems. In the case of CHITOSAN the polyelectrolyte complex is dependent on the pH (pKa = 6.8). At pH = 7.4 the amino group of the chitosan is partially protonated and strongly interacting with the bemiparin. In the PLGA, being a biodegradable copolymer, there is also a process of degradation of the copolymer in addition to the diffusion process, so that after 30 days of release, an increase in bemiparin released in the medium is observed that is not observed in the other systems, reaching 100% release of encapsulated bemiparin at 70 days (Fig. 25).

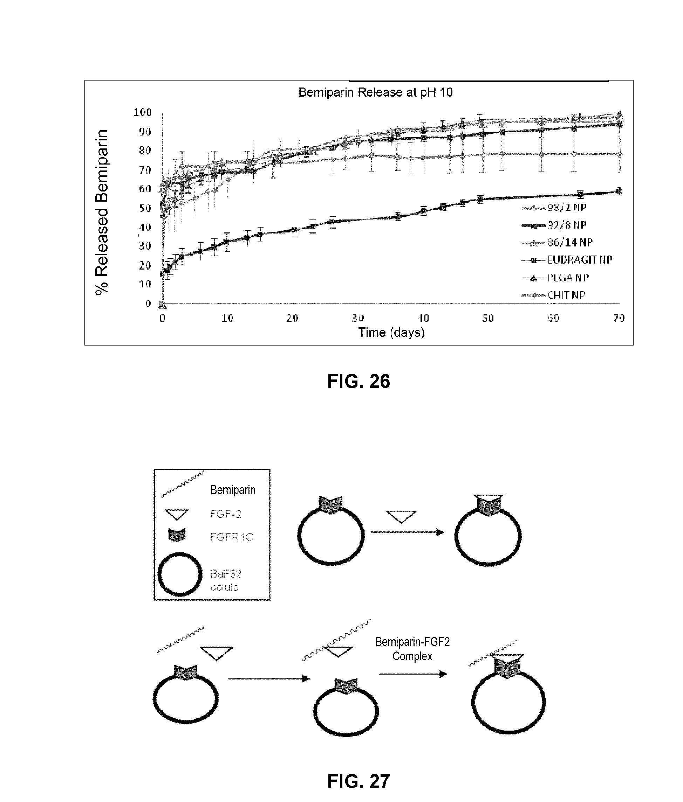

At pH= 10, the hydrolysis of the ester groups is promoted, whereby the process of degradation of the polymeric carrier is accelerated facilitating the diffusion of bemiparin through the nanoparticulate system, thereby obtaining a faster release profile of the same and greater release than that obtained at pH = 7.4 in all the systems. Furthermore, in the case of the chitosan-bemiparin system, the release of the drug increases at pH = 10 due to the rupture of the polyelectrolyte complex, as the amino group is completely deprotonated and does not interact ionically with the bemiparin at this pH, so that its release into the medium is favoured (Fig. 26).

BaF32 Proliferation Assay.

Bemiparin forms a ternary complex (Fig. 27) with the fibroblast growth factor (FGF-2) and its corresponding specific membrane receptor (FGFR1c) thus increasing the cellular signalling process corresponding to the proliferation process (Dillen, K., J. Vandervoort, G. Van den Mooter, and A. Ludwig. 2006. Evaluation of ciprofloxacin-loaded Eudragit® RS100 or RL100/PLGA nanoparticles. International Journal of Pharmaceutics 314 (1): 72-82; Eto, T. 2001. A review of the biological properties and clinical implications of adrenomedullin and proadrenomedullin N-terminal 20 peptide (PAMP), hypotensive and vasodilating peptides. Peptides 22 (11) : 1693-1711). Given this property, an experiment was designed to verify that the encapsulated bemiparin maintains its biological activity. For this BaF32 cells were used, which are a type of lymphocyte B, the cell signalling of which is modulated by a by a specific bond between the FGF-2 and its corresponding FGFR1c receptor on the cell surface. The interaction of the bemiparin with the FGF-2 activates the specific binding of this complex with the FGFR1c receptor, and this results in an increase in cell proliferation due to the specific interaction of bemiparin with the FGF-2 and its corresponding membrane receptor.

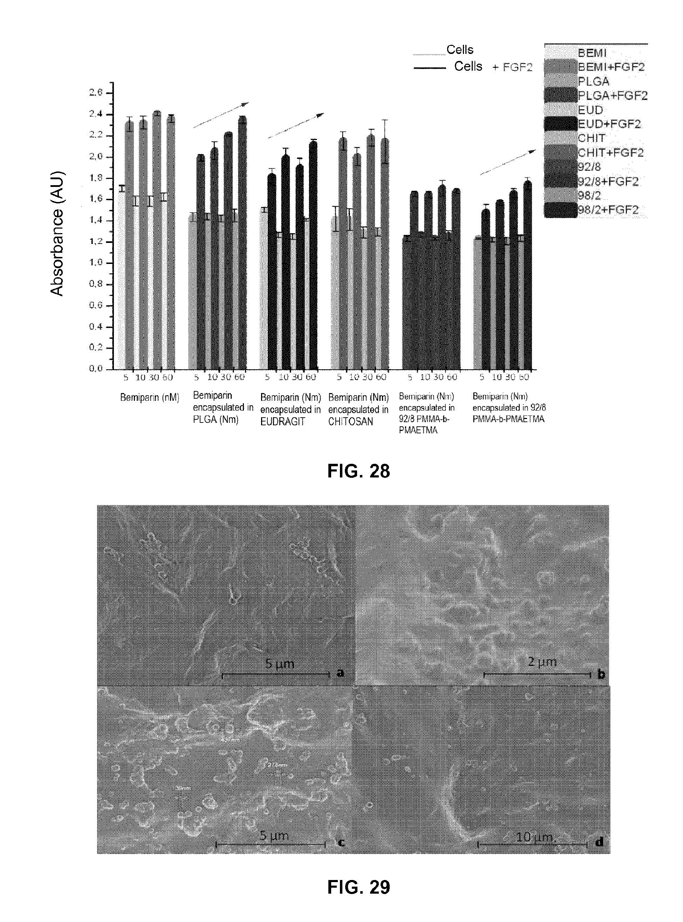

The unencapsulated bemiparin increases the activity of the FGF-2, promoting cell proliferation. This response is independent from the concentration of bemiparin used. However, when the bemiparin is added encapsulated in nanoparticles a certain dependence of the cellular response on the concentration is observed, indicating that the encapsulated bemiparin is being gradually released over time. This effect was mainly observed in the PLGA system and the copolymer 98-2 p(MMA-b-MAETMA) (Fig. 28), where the ionic retention of bemiparin is lower and the process of diffusion of the drug through the polymer matrix is more favoured.

***Example 5. Formation of the two-layer system forming the medical product object of the invention***

The medical product is formed by two biodegradable polymeric layers of different structure and nature. Both layers may contain bioactive components selected according to their application, so that their release is controlled in a programmed manner.

A dispersion or solution of the bioactive component or nanoparticles loaded with the component itself is added to any of them, so that it is physically integrated in the biodegradable polymer matrix.

a) Incorporation of the nanoparticles loaded with bemiparin on the polyurethane film.

An aqueous dispersion of the nanoparticles (0.6 ml, 5 mg/ml) was added on a SPU321-LDI-[50PCL-diol-50PL61] polyurethane film with a mass of 60 mg and 20 mm in diameter, and kept for 24 hrs controlling the saturation of atmospheric water for promoting the diffusion of the particles through the film. Finally the solvent was allowed to evaporate at room temperature. The films loaded with the nanoparticles were analyzed by scan electron microscopy (SEM). Images of the top and bottom surface, as well as of the cross section of the polyurethane film were obtained. In order to make said cross section, the film was immersed in liquid nitrogen and fractured next. The images obtained show that the nanoparticles diffuse through the polyurethane film with an even distribution, keeping their spherical morphology and their initial size (Fig. 29).

b) Incorporation of the hydrogels loaded with PAMP on the polyurethane film loaded with the nanoparticles.

The gelatin-sodium hyaluronate hydrogel cross linked with 1% genipin was hydrated with PBS (pH= 7.4) for 2 hrs and deposited on the polyurethane film loaded with the nanoparticles of bemiparin. A PAMP aqueous solution (0.5 ml, $2x10^6$ M) was added on the hydrogel and the two-layer system was kept for 24 hrs controlling the saturation of the atmospheric water for promoting the diffusion of the PAMP in the hydrogel. The system was stored between two layers of parafilm and sealed to prevent the total evaporation of the solvent. Three types of samples were prepared (Table 8):

> CONTROL DISC: two-layers formed by the polyurethane (SPU) and the Hydrogel (HG). This sample contains no nanoparticles loaded with bemiparin or PAMP.

> DISC LOADED WITH BEMIPARIN: two-layers formed by the polyurethane layer loaded with 5% w of nanoparticles containing bemiparin and the hydrogel layer without loading.

> DISC LOADED WITH BEMIPARIN AND PAMP: two-layers formed by the polyurethane layer loaded with 5% w of nanoparticles containing bemiparin and the hydrogel layer loaded with 1 nanomol of PAMP.

*Table 8. Mass of each of the components formina the two-laver system.*

| SAMPLE | Mass (mg) PU | Mass (mg) NP | Mass (mg) bemiparin | IU bemiparin / disc | Moles (nmol) PAMP / disc |
|---|---|---|---|---|---|
| *SPU+HG CONTROL* | 60 | 0 | 0 | 0 | 0 |
| *SPU + NP + BEMI + HG* | 60 | 3 | 0.3 | 33.4 | 0 |
| *SPU + NP + BEMI + HG + PAMP* | 60 | 3 | 0.3 | 33.4 | 1 |

### Example 6. In vivo assays of the two-layer system: mouse animal model

The three types of two-layer membranes prepared (Table 8) were placed in a pocket formed in the dorsal dermis of genetically modified mice expressing the green fluorescent protein (GFP) in the blood vessels. After 5 days, the membranes were removed and fixed in buffered formalin. Photographs were made of the membranes with a magnifying glass equipped with ultraviolet light and the following results were obtained (Fig. 30a and 30b). The difference between a double disc without PAMP (left micrograph) wherein no blood vessels are observed, and one with PAMP (right micrograph) wherein the neo-formation of vessels blood is observed, was observed.

The images of optical microscopy with hematoxylin-eosin staining correspond to the discs removed after 14 days of implantation and included in resin. Increased formation of blood vessels is observed in the area of the implant containing the discs loaded with PAMP (Fig. 30d).

### Example 7. In vivo assays of the two-layer system: model of experimental ischemia in rabbits

In order to assess the effects on wound healing of the three two-layer systems, an experimental model of excisional defect healing with ischemia in ear in New Zealand white rabbit was used. The animals were split into two groups: ischemic and normoxic (control). The ischemia was carried out by ligation and severing of the central artery and vein of both ears, 24 hours before the intervention. Excisional skin defects were made on the back of each of the ears, with a diameter of 2 cm. Immediately afterwards, the corresponding two-layer systems were applied, which were immobilized using nonabsorbable suture attached to the ends of the wound, following the diagonals of a regular pentagon forming a five-pointed star (Fig. 31).

Fourteen days after the intervention, the animals were killed and samples were taken for histological and morphometric analysis.

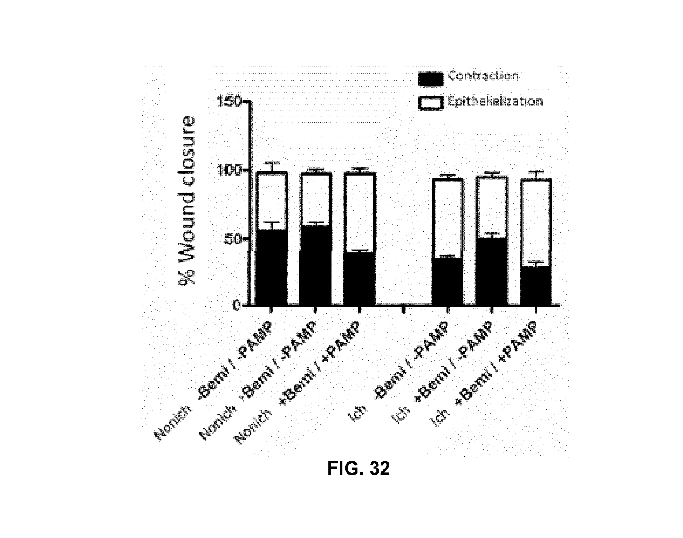

The percentage of closure of the defect was similar in all the groups, although the proportional contribution of the processes of contraction and reepithelialisation showed differences. The systems loaded with Bemiparin and PAMP triggered a lower contraction of the wound, favouring the closing of the same by epithelialisation, particularly in the ischemic group (Fig. 32).

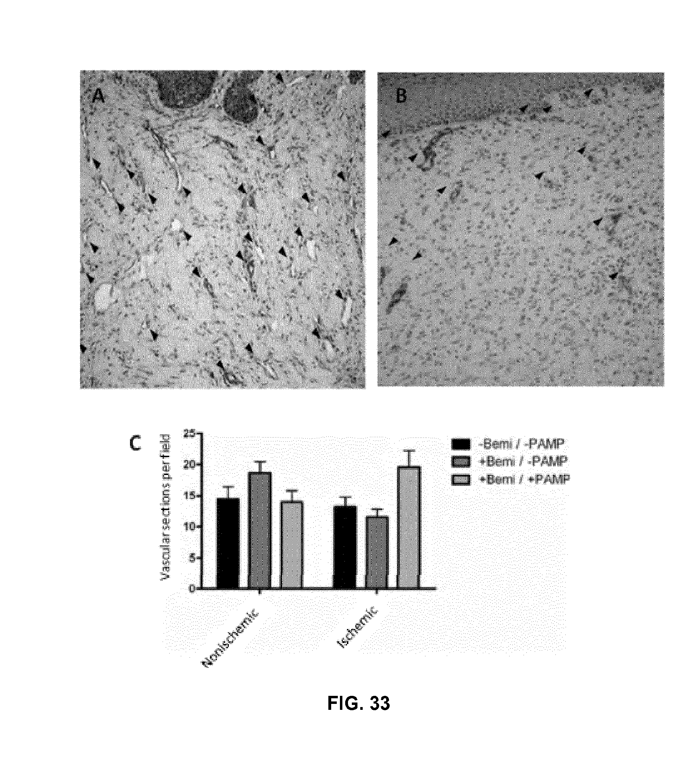

The angiogenic capacity of the two-layer systems was assessed by immunohistochemistry against $\alpha$-smooth muscle actin (ASMA) on sections of the scar tissue. With these samples, counts of the vascular sections observed in the newly formed dermis were made by optical microscopy in fields at 200x magnification. An increase in the number of sections in the samples relating to the systems loaded with Bemiparin and PAMP (Fig. 33) was observed.

## BIBLIOGRAPHIC REFERENCES

Bergmann, A., N. Morgenthaler, J. Papassotiriou, and J. Struck. 2008. diagnosis and assessment of risk for sequellae of diabetes mellitus using fragment of proadrenomedulin, 21 pp.

Bos, G. W., N. M. Scharenborg, A. A. Poot, G. H. M. Engbers, T. Beugeling, W. G. Van Aken, and J. Feijen. 1999.

Proliferation of endothelial cells on surface-immobilized albumin- heparin conjugate loaded with basic fibroblast growth factor. Journal of Biomedical Materials Research 44 (3): 330-340.

Capila, I., and R. J. Linhardt. 2002. Heparin-Protein Interactions. Angewandte Chemie International Edition 41 (3): 390-412.

Ceballos, A., M. Cirri, F. Maestrelli, G. Corti, and P. Mura. 2005. Influence of formulation and process variables on in vitro release of theophylline from directly-compressed Eudragit matrix tablets. II Farmaco 60 (11-12): 913-918.

Cervantes-Uc, J. M., J. I. M. Espinosa, J. V. Cauich-Rodriguez, A. Avila-Ortega, H. Vázquez-Torres, A. Marcos-Fernández, and J. San Roman. 2009. TGA/FTIR studies of segmented aliphatic polyurethanes and their nanocomposites prepared with commercial montmorillonites. Polymer Degradation and Stability 94 (10): 1666-1677.

Collins, M. N., and C. Birkinshaw. 2008. Physical properties of cross linked hyaluronic acid hydrogels. Journal of Materials Science: Materials in Medicine 19 (11): 3335-3343.

Chung, Y.-I., G. Tae, and S. Hong Yuk. 2006. A facile method to prepare heparin-functionalized nanoparticles for controlled release of growth factors. Biomaterials 27 (12): 2621-2626.

Dillen, K., J. Vandervoort, G. Van den Mooter, and A. Ludwig. 2006. Evaluation of ciprofloxacin-loaded Eudragit® RS100 or RL100/PLGA nanoparticles. International Journal of Pharmaceutics 314 (1): 72- 82 .

Eto, T. 2001. A review of the biological properties and clinical implications of adrenomedullin and proadrenomedullin N-terminal 20 peptide (PAMP), hypotensive and vasodilating peptides. Peptides 22 (11): 1693-1711.

Gregory, A., and M. H. Stenzel. 2012. Complex polymer architectures via RAFT polymerization: From fundamental process to extending the scope using click chemistry and nature's building blocks. Progress in Polymer Science (Oxford) 37(1): 38-105.

Han, J., R. W. Cao, B. Chen, L. Ye, A. Y. Zhang, J. Zhang, and Z. G. Feng. 2011. Electrospinning and biocompatibility evaluation of biodegradable polyurethanes based on L -lysine diisocyanate and L-lysine chain extender. Journal of Biomedical Materials Research - Part A 96 A (4): 705-714.

Haslik, W., L. P. Kamolz, G. Nathschlager, H. Andel, G. Meissl, and M. Frey. 2007. First experiences with the collagen-elastin matrix Matriderm® as a dermal substitute in severe burn injuries of the hand. Burns 33 (3): 364-368.

Hoffart, V., A. Lamprecht, P. Maincent, T. Lecompte, C. Vigneron, and N. Ubrich. 2006. Oral bioavailability of a low molecular weight heparin using a polymeric delivery system. Journal of Controlled Release 113(1): 38-42.

Hoffart, V., N. Ubrich, A. Lamprecht, K. Bachelier, C. Vigneron, T. Lecompte, M. Hoffman, and P. Maincent. 2003. Microencapsulation of low molecular weight heparin into polymeric particles designed with biodegradable and nonbiodegradable polycationic polymers. Drug Delivery: Journal of Delivery and Targeting of Therapeutic Agents 10(1): 1-7.

Jiao, Y. Y., N. Ubrich, V. Hoffart, M. Marchand-Arvier, C. Vigneron, M. Hoffman, and P. Maincent. 2002. Preparation and Characterization of Heparin-Loaded Polymeric Microparticles. Drug Development and Industrial Pharmacy 28 (8): 1033-1041.

Jimenez, P. A., and S. E. Jimenez. 2004. Tissue and cellular approaches to wound repair. The American Journal of Surgery 187 (5, Supplement 1): S56-S64.

Lassalle, V., and M. L. Ferreira. 2007. PLA Nano- and Microparticles for Drug Delivery: An Overview of the Methods of Preparation. Macromolecular Bioscience 7 (6): 767-783.

Mao, J. S., H. F. liu, Y. J. Yin, and K. D. Yao. 2003. The properties of chitosan-gelatin membranes and scaffolds modified with hyaluronic acid by different methods. Biomaterials 24 (9): 1621- 1629.

Martinez-Gonzalez, J., and C. Rodriguez. New challenges for a second-generation low-molecular-weight heparin: focus on bemiparin. Expert Rev Cardiovasc Ther 8 (5): 625-634.

Mohammadi, M., S. K. Olsen, and 0. A. Ibrahimi. 2005. Structural basis for fibroblast growth factor receptor activation. Cytokine & Growth Factor Reviews 16 (2) : 107-137.

Moustafine, R. I., I. M. Zaharov, and V. A. Kemenova. 2006. Physicochemical characterization and drug release properties of Eudragit® E PO/Eudragit® L 100-55 interpolyelectrolyte complexes. European Journal of Pharmaceutics and Biopharmaceutics 63 (1) : 26- 36.

Mundargi, R. C., V. R. Babu, V. Rangaswamy, P. Patel, and T. M. Aminabhavi. 2008. Nano/micro technologies for delivering macromolecular therapeutics using poly(d,1-lactide-co-glycolide) and its derivatives. Journal of Controlled Release 125 (3): 193-209.

Pal, K., A. K. Banthia, and D. K. Majumdar. 2007. Preparation and characterization of polyvinyl alcohol-gelatin hydrogel membranes for biomedical applications. AAPS PharmSciTech 8 (1).

Park, S.-N., H. J. Lee, K. H. Lee, and H. Suh. 2003. Biological characterization of EDC-cross linked collagen hyaluronic acid matrix in dermal tissue restoration. Biomaterials 24 (9): 1631-1641.

Peng, H. T., L. Martineau, and P. N. Shek. 2008. Hydrogel-elastomer composite biomaterials: 3. Effects of gelatin molecular weight and type on the preparation and physical properties of interpenetrating polymer networks. Journal of Materials Science: Materials in Medicine 19 (3): 997-1007.

Picard, J., S. Giraudier, and V. Larreta-Garde. 2009. Controlled remodeling of a protein-polysaccharide mixed gel:

examples of gelatin-hyaluronic acid mixtures. Soft Matter 5 (21): 4198-4205 .

Rechichi, A., G. Ciardelli, M. D'Acunto, G. Vozzi, and P. Giusti. 2008. Degradable block polyurethanes from nontoxic building blocks as scaffold materials to support cell growth and proliferation. Journal of Biomedical Materials Research Part A 84A (4): 847-855.

Richard, C., J. P. Liuzzo, and D. Moscatelli. 1995. Fibroblast Growth Factor-2 Can Mediate Cell Attachment by Linking Receptors and Heparan Sulfate Proteoglycans on Neighboring Cells. Journal of Biological Chemistry 270 (41): 24188-24196.

Rios, M. 2003. Micro- and nanoparticle systems show promise for oral dosage forms. Pharmaceutical Technology 27 (2): 17-18.

Rullan, M., L. Cerda, G. Frontera, L. Masmiquel, and J. Llobera. 2008. Treatment of chronic diabetic foot ulcers with bemiparin: a randomized, triple-blind, placebo-controlled, clinical trial. Diabetic Med. 25 (9): 1090-1095.

Stenzel, M. H. 2008. RAFT polymerization: an avenue to functional polymeric micelles for drug delivery. Chemical Communications (30): 3486-3503.

Tanghetti, E., R. Ria, P. Dell'Era, C. Urbinati, M. Rusnati, M. G. Ennas, and M. Presta. 2002. Biological activity of substrate-bound basic fibroblast growth factor (FGF2): Recruitment of FGF receptor- 1 in endothelial cell adhesion contacts. Oncogene 21 (24): 3889- 3897 .

Vanderhooft, J. L., M. Alcoutlabi, J. J. Magda, and G. D. Prestwich. 2009. Rheological Properties of Cross-Linked Hyaluronan-Gelatin Hydrogels for Tissue Engineering. Macromolecular Bioscience 9 (1): 20-28.

Zandi, M., H. Mirzadeh, and C. Mayer. 2007. Effects of concentration, temperature, and pH on chain mobility of gelatin during the early stages of gelation. Iranian Polymer Journal (English Edition) 16 (12): 861-870.

Zeng, C., N. W. Zhang, and J. Ren. 2012. Synthesis and properties of bio-based thermoplastic polyurethane based on poly (L -lactic acid) copolymer polydiol. Journal ofApplied Polymer Science 125 (4) : 2564-2576.

Zhang, H., W. Cui, J. Bei, and S. Wang. 2006. Preparation of poly(lactide-co-glycolide-co-caprolactone) nanoparticles and their degradation behaviour in aqueous solution. Polymer Degradation and Stability 91 (9): 1929-1936.

Zhang, Z., J. Ni, L. Chen, L. Yu, J. Xu, and J. Ding. 2011. Biodegradable and thermoreversible PCLA-PEG-PCLA hydrogel as a barrier for prevention of post-operative adhesion. Biomaterials 32 (21): 4725-4736.

## Claims

1. A dressing **characterised in that** it comprises at least:

   - one inner layer formed by a hydrogel consisting of a combination of sodium hyaluronate and gelatin, and being stabilized with at least one crosslinking agent, and
   - one outer layer formed by a block copolymer type $(A_nB_m)_x(C_oD_p)_y$ which is biodegradable and bioabsorbable polyurethane,

   the inner layer being impregnated with an *N*-terminal peptide of 20 amino acids of pro-adrenomedullin, and the outer layer with nanoparticles of bemiparin encapsulated in a biodegradable copolymer or polymer.

2. Dressing according to the previous claim, wherein the crosslinking agent is selected from the group consisting of genipin, sodium tripolyphosphate, sodium glycerol phosphate and salicin.

3. Dressing according to the previous claim, wherein the crosslinking agent is genipin.

4. Dressing according to any one of the claims 1 to 3, wherein the crosslinking agent is contained in the hydrogel in a concentration comprised between 0.5% and 10% by weight with respect to the polymer mixture, including both limits.

5. Dressing according to any one of the claims 1 to 4, wherein the hydrogel consists of 60% by weight of gelatin and 40% by weight of sodium hyaluronate over the polymeric mass of the hydrogel.

6. Dressing according to any one of the claims 1 to 4, wherein the hydrogel additionally incorporates at least one polymer selected from collagen, chitosan and dextran.

7. Dressing according to any one of the claims 1 to 6, wherein the polyurethane has a molecular weight comprised between 3000 and 40000 Daltons, including both limits.

8. Dressing according to the previous claim, wherein the polyurethane consists of a soft segment $A_nB_m$ formed from

polycaprolactone diol and pluronic 61, a rigid segment $C_oD_p$ consisting of a poly(tetramethylene glycol ether) and L-lysine diisocyanate selected from the group consisting of methyl, ethyl, propyl, isopropyl, butyl and isobutyl.

9. Dressing according to the previous claim, wherein the L-lysine diisocyanate is methyl L-lysine diisocyanate.

10. Dressing according to any one of the claims 1 to 9, wherein the polyurethane is a block copolymer type $(A_nB_m)_x(C_oD_p)_y$ wherein n and m are comprised between 0.2 and 0.8, where m + n = 1 and preferably m=n=0.5, o=2 and p=1.

11. Dressing according to any one of the claims 1 to 10, wherein the polyurethane is a block copolymer of the type $(A_nB_m)_x(C_oD_p)_y$, x and y being comprised between 0.2 and 0.8, where x + y = 1 and preferably x=y=0.5.

12. Dressing according to any one of the claims 1 to 11, wherein when the bemiparin is encapsulated by a polymer, the latter being selected from the group consisting of PLGA, EUDRAGIT and CHITOSAN, and when encapsulated by a copolymer, the latter being a methacrylic amphiphilic block copolymer formed by poly(methyl methacrylate-*b*-[2-(methacryloyloxy)ethyl] trimethylammonium.

13. Dressing according to any one of the claims 1 to 12, wherein the nanoparticles that encapsulate the bemiparin have an average size comprised between 20 and 1000 nm, including both limits.

14. Dressing according to any one of the claims 1 to 13, including one or more additional layers and/or one or more additional active agents.

15. Dressing according to any one of the previous claims, which is selected from the group consisting of a gauze, a bandage and a plaster.

16. A method for the preparation of the dressing described in any one of claims 1 to 15, comprising at least the following steps:

- adding the nanoparticles of bemiparin to the polyurethane layer by impregnation with an aqueous dispersion of said nanoparticles;
- hydrating the hydrogel layer and depositing said layer onto the polyurethane layer previously impregnated with the nanoparticles of bemiparin;
- adding the *N*-terminal peptide of 20 amino acids of pro-adrenomedullin to the hydrogel layer by means of aqueous solution; and
- sealing the product obtained from the two layers.

17. Method according to the previous claim, wherein the aqueous dispersion of nanoparticles has a percentage by weight of said nanoparticles comprised between 1% and 25% by mass with respect to the polymeric mass of polyurethane including both limits, and the aqueous solution containing the N-terminal peptide of 20 amino acids of pro-adrenomedullin and which is added to the hydrogel has a concentration of said peptide comprised between 1 fmol to 1 mmol peptide/disc.

18. Method according to the previous claim, wherein the dressing is sealed after hydration of both layers, by adding an amount of distilled water comprised between 0.2 and 0.5 ml and then contacting said layers keeping them 24 hrs under saturated water vapour atmosphere.

19. Use of a layer formed by a hydrogel consisting of a combination of sodium hyaluronate and gelatin, being cross-linked (stabilized) with at least one crosslinking agent, and which is impregnated with an *N*-terminal peptide of 20 amino acids of pro-adrenomedullin; and a second layer formed by a block copolymer type $(A_nB_m)_x(C_oD_p)_y$ which is biodegradable and bioabsorbable polyurethane containing nanoparticles of bemiparin encapsulated in a biodegradable copolymer or polymer, as described in any one of claims 1 to 14 for the manufacture of a dressing for the use thereof in medicine.

20. Use according to the previous claim, for the treatment and healing of wounds and/or ulcers.

21. A kit for coating skin wounds and ulcers, comprising the dressing described in any one of the claims 1 to 15.

FIG. 1

FIG. 2

**FIG. 3**

**FIG. 4**

**FIG. 5**

**FIG. 6**

PCL-diol

+

PL61

Sn(EtHex)2
24h, 135 °C, DMF

Solft segment

**FIG. 7**

**Rigid segment**

**FIG. 8**

**FIG. 9**

**FIG. 10**

**FIG. 11**

**FIG. 12**

**FIG. 13**

**FIG. 14**

**FIG. 15**

**FIG. 16**

FIG. 17

**FIG. 18**

**FIG. 19**

**FIG. 20**

**FIG. 21**

**FIG. 22**

**FIG. 23**

**FIG. 24**

**FIG. 25**

**FIG. 26**

**FIG. 27**

**FIG. 28**

**FIG. 29**

**FIG. 30**

**FIG. 31**

FIG. 32

FIG. 33

# INTERNATIONAL SEARCH REPORT

| International application No. |
|---|
| PCT/ES2013/070774 |

### A. CLASSIFICATION OF SUBJECT MATTER

**See extra sheet**

According to International Patent Classification (IPC) or to both national classification and IPC

### B. FIELDS SEARCHED

Minimum documentation searched (classification system followed by classification symbols)
A61F, A61L

Documentation searched other than minimum documentation to the extent that such documents are included in the fields searched

Electronic data base consulted during the international search (name of data base and, where practicable, search terms used)

EPODOC, INVENES, WPI, MEDLINE, NPL, EMBASE, BIOSIS, XPESP, XPESP2, XPOAC

### C. DOCUMENTS CONSIDERED TO BE RELEVANT

| Category* | Citation of document, with indication, where appropriate, of the relevant passages | Relevant to claim No. |
|---|---|---|
| A | Valenta C. et al. The use of polymers for dermal and transdermal delivery. European Journal of Pharmaceutics and Biopharmaceutics. 09.2004, Vol. 58, N° 2 , pages 279-289 (the whole document) | 1-21 |
| A | US 5204110 A  (CARTMELL ET AL) 20.04.1993, (the whole document) | 1-21 |
| A | TW 200938234 A (TEIKOKU SEIYAKU KK) 16.09.2009 (abstract) Derwent Publications Ltd., AN 2008-F67177 | 1-21 |

☐ Further documents are listed in the continuation of Box C.　　　　☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 29/01/2014 | **(03/02/2014)** |

| Name and mailing address of the ISA/　　　　　　　　　　OFICINA ESPAÑOLA DE PATENTES Y MARCAS Paseo de la Castellana, 75 - 28071 Madrid (España) Facsimile No.: 91 349 53 04 | Authorized officer M. Cumbreño Galindo　　　　　　　　　　Telephone No. 91 3496880 |
|---|---|

Form PCT/ISA/210 (second sheet) (July 2009)

## INTERNATIONAL SEARCH REPORT

Information on patent family members

| | International application No. |
| --- | --- |
| | PCT/ES2013/070774 |

| Patent document cited in the search report | Publication date | Patent family member(s) | Publication date |
| --- | --- | --- | --- |
| US5204110 A | 20.04.1993 | JPH04227259 A | 17.08.1992 |
| | | JPH0724671B B2 | 22.03.1995 |
| | | EP0455324 A1 | 06.11.1991 |
| | | EP0455324 B1 | 07.06.1995 |
| | | DE69110193T T2 | 19.10.1995 |
| | | AT123402T T | 15.06.1995 |
| | | AU7026991 A | 07.11.1991 |
| | | AU620750B B2 | 20.02.1992 |
| | | NZ237005 A | 28.04.1993 |
| | | CA2035490 A1 | 03.11.1991 |
| | | CA2035490 C | 09.01.2001 |
| | | US5115801 A | 26.05.1992 |
| TW200938234 A | 16.09.2009 | MX2010009302 A | 24.09.2010 |
| | | US2010324464 A1 | 23.12.2010 |
| | | US8563799 B2 | 22.10.2013 |
| | | NZ587365 A | 27.04.2012 |
| | | KR20100126297 A | 01.12.2010 |
| | | WO2009107189 A1 | 03.09.2009 |
| | | EP2246073 A1 | 03.11.2010 |
| | | EP2246073 A4 | 23.01.2013 |
| | | CN101959539 A | 26.01.2011 |
| | | CA2714490 A1 | 03.09.2009 |
| | | AU2008351834 A1 | 03.09.2009 |
| | | AU2008351834B B2 | 12.09.2013 |
| | | JP2008073287 A | 03.04.2008 |
| | | JP5147207B B2 | 20.02.2013 |

Form PCT/ISA/210 (patent family annex) (July 2009)

**EP 2 921 148 A1**

## INTERNATIONAL SEARCH REPORT

International application No.

PCT/ES2013/070774

### CLASSIFICATION OF SUBJECT MATTER

*A61F13/02* (2006.01)
*A61L15/22* (2006.01)
*A61L15/44* (2006.01)

Form PCT/ISA/210 (extra sheet) (July 2009)

43

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03053458 A1
- WO 2011002249 A, Zhang
- WO 2011028031 A
- WO 2010120757 A
- US 20110038921 A1
- ES 2204886 T3

**Non-patent literature cited in the description**

- **CAPILA, I. ; R. J. LINHARDT.** Heparin-Protein Interactions. *Angewandte Chemie International Edition,* 2002, vol. 41 (3), 390-412
- **DILLEN, K. ; J. VANDERVOORT ; G. VAN DEN MOOTER ; A. LUDWIG.** Evaluation of ciprofloxacin-loaded Eudragit® RS100 or RL100/PLGA nanoparticles. *International Journal of Pharmaceutics,* 2006, vol. 314 (1), 72-82
- **ETO, T.** A review of the biological properties and clinical implications of adrenomedullin and proadrenomedullin N-terminal 20 peptide (PAMP), hypotensive and vasodilating peptides. *Peptides,* 2001, vol. 22 (11), 1693-1711
- **BERGMANN, A. ; N. MORGENTHALER ; J. PAPASSOTIRIOU ; J. STRUCK.** *diagnosis and assessment of risk for sequellae of diabetes mellitus using fragment of proadrenomedulin,* 2008, 21
- **BOS, G. W. ; N. M. SCHARENBORG ; A. A. POOT ; G. H. M. ENGBERS ; T. BEUGELING ; W. G. VAN AKEN ; J. FEIJEN.** Proliferation of endothelial cells on surface-immobilized albumin- heparin conjugate loaded with basic fibroblast growth factor. *Journal of Biomedical Materials Research,* 1999, vol. 44 (3), 330-340
- **CAPILA, I. ; R. J. LINHARDT.** Heparin-Protein Interactions. *Angewandte Chemie International Edition,* 2002, vol. 41 (3), 390-412
- **CEBALLOS, A. ; M. CIRRI ; F. MAESTRELLI ; G. CORTI ; P. MURA.** Influence of formulation and process variables on in vitro release of theophylline from directly-compressed Eudragit matrix tablets. *Il Farmaco,* 2005, vol. 60 (11-12), 913-918
- **CERVANTES-UC, J. M. ; J. I. M. ESPINOSA ; J. V. CAUICH-RODRIGUEZ ; A. AVILA-ORTEGA ; H. VÁZQUEZ-TORRES ; A. MARCOS-FERNÁNDEZ ; J. SAN ROMAN.** TGA/FTIR studies of segmented aliphatic polyurethanes and their nanocomposites prepared with commercial montmorillonites. *Polymer Degradation and Stability,* 2009, vol. 94 (10), 1666-1677
- **COLLINS, M. N. ; C. BIRKINSHAW.** Physical properties of cross linked hyaluronic acid hydrogels. *Journal of Materials Science: Materials in Medicine,* 2008, vol. 19 (11), 3335-3343
- **CHUNG, Y.-I. ; G. TAE ; S. HONG YUK.** A facile method to prepare heparin-functionalized nanoparticles for controlled release of growth factors. *Biomaterials,* 2006, vol. 27 (12), 2621-2626
- **DILLEN, K. ; J. VANDERVOORT ; G. VAN DEN MOOTER ; A. LUDWIG.** Evaluation of ciprofloxacin-loaded Eudragit® RS100 or RL100/PLGA nanoparticles. *International Journal of Pharmaceutics,* 2006, vol. 314 (1), 72-82
- **GREGORY, A. ; M. H. STENZEL.** Complex polymer architectures via RAFT polymerization: From fundamental process to extending the scope using click chemistry and nature's building blocks. *Progress in Polymer Science (Oxford),* 2012, vol. 37 (1), 38-105
- **HAN, J. ; R. W. CAO ; B. CHEN ; L. YE ; A. Y. ZHANG ; J. ZHANG ; Z. G. FENG.** Electrospinning and biocompatibility evaluation of biodegradable polyurethanes based on L -lysine diisocyanate and L-lysine chain extender. *Journal of Biomedical Materials Research,* 2011, vol. 96 A (4), 705-714
- **HASLIK, W. ; L. P. KAMOLZ ; G. NATHSCHLAGER ; H. ANDEL ; G. MEISSL ; M. FREY.** First experiences with the collagen-elastin matrix Matriderm® as a dermal substitute in severe burn injuries of the hand. *Burns,* 2007, vol. 33 (3), 364-368
- **HOFFART, V. ; A. LAMPRECHT ; P. MAINCENT ; T. LECOMPTE ; C. VIGNERON ; N. UBRICH.** Oral bioavailability of a low molecular weight heparin using a polymeric delivery system. *Journal of Controlled Release,* 2006, vol. 113 (1), 38-42

- **HOFFART, V. ; N. UBRICH ; A. LAMPRECHT ; K. BACHELIER ; C. VIGNERON ; T. LECOMPTE ; M. HOFFMAN ; P. MAINCENT.** Microencapsulation of low molecular weight heparin into polymeric particles designed with biodegradable and nonbiodegradable polycationic polymers. *Drug Delivery: Journal of Delivery and Targeting of Therapeutic Agents,* 2003, vol. 10 (1), 1-7
- **JIAO, Y. Y. ; N. UBRICH ; V. HOFFART ; M. MARCHAND-ARVIER ; C. VIGNERON ; M. HOFFMAN ; P. MAINCENT.** Preparation and Characterization of Heparin-Loaded Polymeric Microparticles. *Drug Development and Industrial Pharmacy,* 2002, vol. 28 (8), 1033-1041
- **JIMENEZ, P. A. ; S. E. JIMENEZ.** Tissue and cellular approaches to wound repair. *The American Journal of Surgery,* 2004, vol. 187 (5), S56-S64
- **LASSALLE, V. ; M. L. FERREIRA.** PLA Nano- and Microparticles for Drug Delivery: An Overview of the Methods of Preparation. *Macromolecular Bioscience,* 2007, vol. 7 (6), 767-783
- **MAO, J. S. ; H. F. LIU ; Y. J. YIN ; K. D. YAO.** The properties of chitosan-gelatin membranes and scaffolds modified with hyaluronic acid by different methods. *Biomaterials,* 2003, vol. 24 (9), 1621-1629
- **MARTINEZ-GONZALEZ, J. ; C. RODRIGUEZ.** New challenges for a second-generation low-molecular-weight heparin: focus on bemiparin. *Expert Rev Cardiovasc Ther,* vol. 8 (5), 625-634
- **MOHAMMADI, M. ; S. K. OLSEN ; 0. A. IBRAHIMI.** Structural basis for fibroblast growth factor receptor activation. *Cytokine & Growth Factor Reviews,* 2005, vol. 16 (2), 107-137
- **MOUSTAFINE, R. I. ; I. M. ZAHAROV ; V. A. KEMENOVA.** Physicochemical characterization and drug release properties of Eudragit® E PO/Eudragit® L 100-55 interpolyelectrolyte complexes. *European Journal of Pharmaceutics and Biopharmaceutics,* 2006, vol. 63 (1), 26-36
- **MUNDARGI, R. C. ; V. R. BABU ; V. RANGASWAMY ; P. PATEL ; T. M. AMINABHAVI.** Nano/micro technologies for delivering macromolecular therapeutics using poly(d,1-lactide-co-glycolide) and its derivatives. *Journal of Controlled Release,* 2008, vol. 125 (3), 193-209
- **PAL, K. ; A. K. BANTHIA ; D. K. MAJUMDAR.** Preparation and characterization of polyvinyl alcohol-gelatin hydrogel membranes for biomedical applications. *AAPS PharmSciTech,* 2007, vol. 8 (1
- **PARK, S.-N. ; H. J. LEE ; K. H. LEE ; H. SUH.** Biological characterization of EDC-cross linked collagen hyaluronic acid matrix in dermal tissue restoration. *Biomaterials,* 2003, vol. 24 (9), 1631-1641
- **PENG, H. T. ; L. MARTINEAU ; P. N. SHEK.** Hydrogel-elastomer composite biomaterials: 3. Effects of gelatin molecular weight and type on the preparation and physical properties of interpenetrating polymer networks. *Journal of Materials Science: Materials in Medicine,* 2008, vol. 19 (3), 997-1007
- **PICARD, J. ; S. GIRAUDIER ; V. LARRETA-GARDE.** Controlled remodeling of a protein-polysaccharide mixed gel: examples of gelatin-hyaluronic acid mixtures. *Soft Matter,* 2009, vol. 5 (21), 4198-4205
- **RECHICHI, A. ; G. CIARDELLI ; M. D'ACUNTO ; G. VOZZI ; P. GIUSTI.** Degradable block polyurethanes from nontoxic building blocks as scaffold materials to support cell growth and proliferation. *Journal of Biomedical Materials Research,* 2008, vol. 84A (4), 847-855
- **RICHARD, C. ; J. P. LIUZZO ; D. MOSCATELLI.** Fibroblast Growth Factor-2 Can Mediate Cell Attachment by Linking Receptors and Heparan Sulfate Proteoglycans on Neighboring Cells. *Journal of Biological Chemistry,* 1995, vol. 270 (41), 24188-24196
- **RIOS, M.** Micro- and nanoparticle systems show promise for oral dosage forms. *Pharmaceutical Technology,* 2003, vol. 27 (2), 17-18
- **RULLAN, M ; L. CERDA ; G. FRONTERA ; L. MASMIQUEL ; J. LLOBERA.** Treatment of chronic diabetic foot ulcers with bemiparin: a randomized, triple-blind, placebo-controlled, clinical trial. *Diabetic Med.,* 2008, vol. 25 (9), 1090-1095
- **STENZEL, M. H.** RAFT polymerization: an avenue to functional polymeric micelles for drug delivery. *Chemical Communications,* 2008, 3486-3503
- **TANGHETTI, E. ; R. RIA ; P. DELL'ERA ; C. URBINATI ; M. RUSNATI ; M. G. ENNAS ; M. PRESTA.** Biological activity of substrate-bound basic fibroblast growth factor (FGF2): Recruitment of FGF receptor- 1 in endothelial cell adhesion contacts. *Oncogene,* 2002, vol. 21 (24), 3889-3897
- **VANDERHOOFT, J. L. ; M. ALCOUTLABI ; J. J. MAGDA ; G. D. PRESTWICH.** Rheological Properties of Cross-Linked Hyaluronan-Gelatin Hydrogels for Tissue Engineering. *Macromolecular Bioscience,* 2009, vol. 9 (1), 20-28
- **ZANDI, M. ; H. MIRZADEH ; C. MAYER.** Effects of concentration, temperature, and pH on chain mobility of gelatin during the early stages of gelation. *Iranian Polymer Journal,* 2007, vol. 16 (12), 861-870
- **ZENG, C. ; N. W. ZHANG ; J. REN.** Synthesis and properties of bio-based thermoplastic polyurethane based on poly (L -lactic acid) copolymer polydiol. *Journal ofApplied Polymer Science,* 2012, vol. 125 (4), 2564-2576

- **ZHANG, H. ; W. CUI ; J. BEI ; S. WANG.** Preparation of poly(lactide-co-glycolide-co-caprolactone) nanoparticles and their degradation behaviour in aqueous solution. *Polymer Degradation and Stability,* 2006, vol. 91 (9), 1929-1936

- **ZHANG, Z. ; J. NI ; L. CHEN ; L. YU ; J. XU ; J. DING.** Biodegradable and thermoreversible PCLA-PEG-PCLA hydrogel as a barrier for prevention of post-operative adhesion. *Biomaterials,* 2011, vol. 32 (21), 4725-4736